(19) Europäisches Patentamt
European Patent Office
Office européen des brevets

(11) EP 2 588 438 B1

(12) **EUROPÄISCHE PATENTSCHRIFT**

(45) Veröffentlichungstag und Bekanntmachung des Hinweises auf die Patenterteilung:
**08.04.2015 Patentblatt 2015/15**

(21) Anmeldenummer: **11726411.9**

(22) Anmeldetag: **16.06.2011**

(51) Int Cl.:
***C07C 51/00*** *(2006.01)* ***C07C 53/02*** *(2006.01)*

(86) Internationale Anmeldenummer:
**PCT/EP2011/060012**

(87) Internationale Veröffentlichungsnummer:
**WO 2012/000799 (05.01.2012 Gazette 2012/01)**

(54) **VERFAHREN ZUR HERSTELLUNG VON AMEISENSÄURE DURCH UMSETZUNG VON KOHLENDIOXID MIT WASSERSTOFF**

PROCESS FOR THE PREPARATION OF FORMIC ACID BY REACTING CARBON DIOXIDE AND HYDROGEN

PROCÉDÉ DE PRODUCTION D'ACIDE FORMIQUE PAR MISE EN RÉACTION DE DIOXYDE DE CARBONE AVEC DE L'HYDROGÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.06.2010 EP 10167709**

(43) Veröffentlichungstag der Anmeldung:
**08.05.2013 Patentblatt 2013/19**

(73) Patentinhaber: **BASF SE**
**67056 Ludwigshafen (DE)**

(72) Erfinder:
- **SCHAUB, Thomas**
  **67433 Neustadt (DE)**
- **FRIES, Donata Maria**
  **68199 Mannheim (DE)**
- **PACIELLO, Rocco**
  **67098 Bad Dürkheim (DE)**
- **MOHL, Klaus-Dieter**
  **68766 Hockenheim (DE)**
- **SCHÄFER, Martin**
  **67269 Grünstadt (DE)**
- **RITTINGER, Stefan**
  **68199 Mannheim (DE)**
- **SCHNEIDER, Daniel**
  **68159 Mannheim (DE)**

(56) Entgegenhaltungen:
**WO-A1-86/02066** **WO-A1-2008/116799**
**DE-T2- 68 920 933**

EP 2 588 438 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft ein Verfahren zur Herstellung von Ameisensäure durch Umsetzung von Kohlendioxid mit Wasserstoff in einem Hydrierreaktor in Gegenwart eines Katalysators, enthaltend ein Element aus der 8, 9. oder 10. Gruppe des Periodensystems, eines tertiären Amins und eines polaren Lösungsmittels, unter Bildung von Ameisensäure/Amin-Addukten als Intermediaten, die anschließend thermisch gespalten werden.

Addukte aus Ameisensäure und tertiären Aminen können thermisch in freie Ameisensäure und tertiäres Amin gespalten werden und dienen daher als Intermediate in der Herstellung von Ameisensäure (DE 68 9 20 933 T2).

Ameisensäure ist ein wichtiges und vielseitig einsetzbares Produkt. Sie wird beispielsweise zum Ansäuern bei der Herstellung von Futtermitteln, als Konservierungsmittel, als Desinfektionsmittel, als Hilfsstoff in der Textil- und Lederindustrie, als Gemisch mit ihren Salzen zur Enteisung von Flugzeugen und Start- und Landebahnen sowie als Synthesebaustein in der chemischen Industrie verwendet.

Die genannten Addukte aus Ameisensäure und tertiären Aminen können auf verschiedene Art und Weise hergestellt werden, beispielsweise (i) durch direkte Umsetzung des tertiären Amins mit Ameisensäure, (ii) durch Hydrolyse von Methylformiat zu Ameisensäure in Gegenwart des tertiären Amins oder (iii) durch katalytische Hydratisierung von Kohlenmonoxid oder Hydrierung von Kohlendioxid zu Ameisensäure in Gegenwart des tertiären Amins. Das letztgenannte Verfahren der katalytischen Hydrierung von Kohlendioxid hat den besonderen Vorteil, dass Kohlendioxid in großen Mengen verfügbar und hinsichtlich seiner Quelle flexibel ist.

Großtechnisch aussichtsreich erscheint dabei vor allem die katalytische Hydrierung von Kohlendioxid in Gegenwart von Aminen (W. Leitner, Angewandte Chemie 1995, 107, Seiten 2391 bis 2405; P. G. Jessop, T. Ikariya, R. Noyori, Chemical Reviews 1995, 95, Seiten 259 bis 272). Die hierbei gebildeten Addukte aus Ameisensäure und Aminen lassen sich thermisch in Ameisensäure und das eingesetzte, in die Hydrierung rückführbare Amin, spalten.

Der für die Reaktion notwendige Katalysator enthält ein oder mehrere Elements aus der 8., 9. oder 10. Gruppe des Periodensystems, also Fe, Co, Ni, Ru, Rh, Pd, Os, Ir und/oder Pt. Bevorzugt enthält der Katalysator Ru, Rh, Pd, Os, Ir und/oder Pt, besonders bevorzugt Ru, Rh und/oder Pd und ganz besonders bevorzugt Ru.

Um ein wirtschaftliches Verfahren zu ermöglichen, muss der verwendete Katalysator aus zwei Gründen möglichst vollständig aus dem Produktstrom abgetrennt und in die Reaktion zurückgeführt werden:

(1) große Verluste des teuren Katalysators würden erhebliche Zusatzkosten verursachen und wären für einen wirtschaftlichen Betrieb des Verfahrens prohibitiv.
(2) bei der thermischen Spaltung der Ameisensäure/Amin-Addukte sollte möglichst wenig Katalysator vorliegen, da dieser unter Abwesenheit eines $CO_2$- und/oder

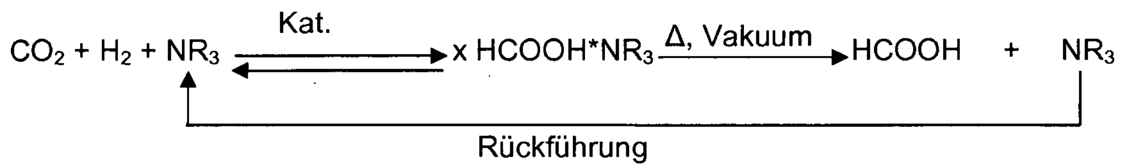


$H_2$-Druckes auch die Rückreaktion katalysiert und damit zu Verlusten an der gebildeten Ameisensäure führt.

Bildung von Ameisensäure durch $CO_2$-Hydrierung (x = 0.4 - 3)

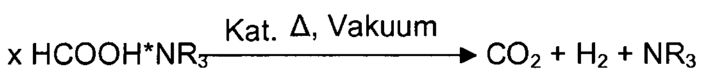


Zersetzung von Ameisensäure/Amin-Addukten unter Anwesenheit eines Katalysators

(x = 0.4 – 3)

Die Übergangsmetall-katalysierte Zersetzung von Ameisensäure wurde vor allem in letzter Zeit ausführlich beschrieben: C. Fellay, N. Yan, P.J. Dyson, G. Laurenczy Chem. Eur. J. 2009, 15, 3752-3760; C. Fellay, P.J. Dyson, G. Laurenczy Angew. Chem. 2008, 120, 4030-4032; B. Loges, A. Boddien, H. Junge, M. Beller Angew. Chem. 2008, 120, 4026-4029; F. Joó ChemSusChem 2008, 1, 805-808; S. Enthaler ChemSusChem 2008, 1, 801-804; S. Fukuzumi, T. Kobayashi, T. Suenobu ChemSusChem 2008, 1, 827-834;A. Boddien, B. Loges, H. Junge, M. Beller ChemSusChem

2008, 1, 751-758.

Die hierbei eingesetzten Katalysatoren eignen sich grundsätzlich auch zur Hydrierung von $CO_2$ zu Ameisensäure (P.G. Jessop, T. Ikariya, R. Noyori Chem. Rev. 1995, 95, 259-272; P.G. Jessop, F. Joó, C.C. Tai Coord. Chem. Rev. 2004, 248, 2425-2442; P.G. Jessop, Homogeneous Hydrogenation of Carbon Dioxide, in: The Handbook of Homogeneous Hydrogenation, Hrsg.: J.G. de Vries, C.J. Elsevier, Volume 1, 2007, Wiley-VCH, S. 489-511). Somit müssen die Hydrierkatalysatoren vor der thermischen Spaltung abgetrennt werden, um die unerwünschte Ameisensäurezersetzung zu unterbinden.

WO 2008/116.799 offenbart ein Verfahren zur Hydrierung von Kohlendioxid in Gegenwart eines in Lösung suspendierten oder homogen gelösten Katalysators enthaltend ein Übergangsmetall der VIII. Nebengruppe (Gruppe 8, 9, 10), eines tertiären Amins mit mindestens einer Hydroxylgruppe und eines polaren Lösungsmittels zu einem Addukt aus Ameisensäure und dem tertiären Amin. Durch die Hydroxylgruppe(n) im tertiären Amin wird gegenüber dem sonst üblicherweise eingesetzten Triethylamin eine erhöhte Kohlendioxid-Löslichkeit erreicht. Als bevorzugte Homogenkatalysatoren sind $RuH_2L_4$ mit einzähnigen Phosphor-basierten Liganden L und $RuH_2(LL)_2$ mit zweizähnigen Phosphor-basierten Liganden LL und als besonders bevorzugt $RuH_2[P(C_6H_5)_3]_4$ genannt. Als polare Lösungsmittel sind Alkohole, Ether, Sulfolane, Dimethylsulfoxid und Amide, deren Siedepunkt bei Normaldruck mindestens 5°C oberhalb dem von Ameisensäure liegt, genannt. Auch die bevorzugt einzusetzenden tertiären Amine weisen einen Siedepunkt oberhalb dem der Ameisensäure auf. Da keine Phasentrennung stattfindet, erfolgt die Aufarbeitung des gesamten Reaktionsaustrags durch Destillation, gegebenenfalls nach vorheriger Abtrennung des Katalysators, wobei dabei das gebildete Addukt aus Ameisensäure und dem tertiären Amin thermisch gespalten und die freigesetzte Ameisensäure als Kopfprodukt gewonnen wird. Das Sumpfprodukt enthaltend tertiäres Amin, polares Lösungsmittel und gegebenenfalls Katalysator wird zur Hydrierstufe zurückgeführt.

Nachteilig an diesem Verfahren ist die Zuführung des gesamten flüssigen Reaktionsaustrags in die Vorrichtung zur thermischen Spaltung und Destillation, gegebenenfalls nach vorgeschalteter spezifischer Abtrennung des homogenen Katalysators durch einen separaten Verfahrensschritt wie beispielsweise einer Extraktions-, Adsorptions- oder Ultrafiltrationsstufe. Als Folge davon ist die Vorrichtung zur thermischen Spaltung und Destillation hinsichtlich der höheren Flüssigkeitsbelastung als auch hinsichtlich der spezifischeren Trenneigenschaften größer und komplexer auszulegen, was sich unter anderem auch in den Investitionskosten niederschlägt (zum Beispiel via Ingenieurleistung, Material, Flächenbedarf). Zudem bedingt die höhere Flüssigkeitsbelastung auch einen höheren Energiebedarf.

Die grundlegenden Arbeiten zur katalytische Hydrierung von Kohlendioxid zu Ameisensäure wurden jedoch bereits in den 1970er und 1980er Jahren gemacht. Als Ausfluss davon dürften wohl auch die in EP 0 095 321 A, EP 0 151 510 A und EP 0 181 078 A zum Patent angemeldeten Verfahren der BP Chemicals Ltd zu verstehen sein. Alle drei Schriften beschreiben die Hydrierung von Kohlendioxid in Gegenwart eines homogenen Katalysators enthaltend ein Übergangsmetall der VIII. Nebengruppe (Gruppe 8, 9, 10), eines tertiären Amins und eines polaren Lösungsmittels zu einem Addukt aus Ameisensäure und dem tertiären Amin. Als bevorzugte Homogenkatalysatoren sind in EP 0 095 321 A und EP 0 181 078 A jeweils Ruthenium-basierte Carbonyl-, Halogenid- und/oder Triphenylphosphin-haltige Komplexkatalysatoren und in EP 0 151 510 A Rhodium/Phosphin-Komplexe genannt. Bevorzugte tertiäre Amine sind $C_1$-$C_{10}$-Trialkylamine, insbesondere die kurzkettigen $C_1$-$C_4$-Trialkylamine, sowie cyclische und/oder verbrückte Amine wie 1,8-Diazabicyclo[5.4.0]undec-7-en, 1,4-Diazabicyclo[2.2.2]octan, Pyridin oder Picoline. Die Hydrierung erfolgt bei einem Kohlendioxid-Partialdruck von bis zu 6 MPa (60 bar), einem Wasserstoff-Partialdruck von bis zu 25 MPa (250 bar) und einer Temperatur von etwa Raumtemperatur bis 200°C.

EP 0 095 321 A und EP 0 151 510 A lehren den Einsatz eines Alkohols als polares Lösungsmittel. Da jedoch primäre Alkohole zur Bildung von Ameisensäureestern (organischen Formiaten) neigen, sind sekundäre Alkohole, insbesondere Isopropanol, bevorzugt. Zudem wird die Gegenwart von Wasser als vorteilhaft beschrieben. Gemäß den Beispielen aus EP 0 095 321 A erfolgt die Aufarbeitung des Reaktionsaustrags durch eine direkt nachfolgende zweistufige Destillation, bei der in der ersten Stufe die Leichtsieder Alkohol, Wasser, tertiäres Amin und in der zweiten Stufe unter Vakuumbedingungen das Addukt aus Ameisensäure und dem tertiären Amin über Kopf abgetrennt werden. EP 0 151 510 A lehrt ebenfalls eine destillative Aufarbeitung, jedoch unter Verweis auf EP 0 126 524 A mit nachfolgendem Austausch des tertiären Amins im destillativ abgetrennten Addukt vor dessen thermischer Spaltung durch eine schwächere, weniger flüchtige Stickstoffbase, um die nachfolgende thermische Spaltung zur Darstellung der freien Ameisensäure zu erleichtern, beziehungsweise erst möglich zu machen.

EP 0 181 078 A lehrt die gezielte Auswahl des polaren Lösungsmittels anhand von drei wesentlichen Kriterien, die gleichzeitig erfüllt sein müssen:

(i) der homogene Katalysator muss im polaren Lösungsmittel löslich sein;
(ii) das polare Lösungsmittel darf die Hydrierung nicht nachteilig beeinflussen; und
(iii) das gebildete Addukt aus Ameisensäure und dem tertiären Amin sollte leicht vom polaren Lösungsmittel abtrennbar sein.

Als besonders geeignete polare Lösungsmittel sind verschiedene Glykole und Phenylpropanole genannt.

Die Aufarbeitung des Reaktionsaustrags gemäß der Lehre von EP 0 181 078 A erfolgt derart, dass zunächst in einem Verdampfer die gasförmigen Komponenten (vor allem nicht umgesetzte Edukte Wasserstoff und Kohlendioxid) über Kopf und der homogene Katalysator gelöst im polaren Lösungsmittel über Sumpf abgetrennt und zur Hydrierstufe zurückgeführt werden. Aus der verbleibenden Flüssigphase enthaltend das Addukt aus Ameisensäure und dem tertiären Amin, freies tertiäres Amin und gegebenenfalls Wasser wird dann anschließend das Addukt aus Ameisensäure und dem tertiären Amin abgetrennt und der verbleibende Teil der Flüssigphase enthaltend das freie tertiäre Amin und gegebenenfalls Wasser zur Hydrierstufe zurückgeführt. Die Abtrennung kann durch Destillation oder durch Phasentrennung des Zweiphasensystems (Dekantierung) erfolgen.

Eine weitere wesentliche Lehre aus EP 0 181 078 A ist der anschließende, zwingende Austausch des tertiären Amins im abgetrennten Addukt vor dessen thermischer Spaltung durch eine schwächere, weniger flüchtige Stickstoffbase, um die nachfolgende thermische Spaltung zur Darstellung der freien Ameisensäure zu erleichtern beziehungsweise erst möglich zu machen. Als besonders geeignete schwächere Stickstoffbasen sind Imidazolderivate wie beispielsweise 1-n-Butylimidazol genannt.

Nachteilig am Verfahren des EP 0 181 078 A ist die sehr aufwändige, vierstufige Aufarbeitung des Reaktionsaustrags durch

(i) Abtrennung der gasförmigen Komponenten sowie des homogenen Katalysators und des polaren Lösungsmittels in einem Verdampfer und Rückführung zur Hydrierstufe;
(ii) Abtrennung des Addukts aus Ameisensäure und dem tertiären Amin in einer Destillationskolonne oder einem Phasenscheider und Rückführung des verbleibenden Flüssigkeitsstroms zur Hydrierstufe;
(iii) Austausch des tertiären Amins im Addukt aus Ameisensäure und dem tertiären Amin durch eine schwächere, weniger flüchtige Stickstoffbase in einem Reaktionskessel mit aufgesetzter Destillationskolonne und Rückführung des freigesetzten tertiären Amins zur Hydrierstufe; und
(iv) thermische Spaltung des Addukts aus Ameisensäure und der schwächeren Stickstoffbase und Rückführung der freigesetzten schwächeren Stickstoffbase zur Basenaustauschstufe.

Ein weiterer, wesentlicher Nachteil am Verfahren des EP 0 181 078 A, sowie auch an den Verfahren der EP 0 095 321 A und EP 0 151 510 A ist die Tatsache, dass sich das Addukt aus Ameisensäure und dem tertiären Amin in Gegenwart des homogenen Katalysators bei der Aufarbeitung im Verdampfer zum Teil wieder in Kohlendioxid und Wasserstoff rückspaltet. In EP 0 329 337 A ist daher als Lösung des Problems die Zugabe eines Zersetzungsinhibitors vorgeschlagen, welcher den homogenen Katalysator reversibel inhibiert. Als bevorzugte Zersetzungsinhibitoren sind Kohlenmonoxid und Oxidationsmittel genannt. Nachteilig daran ist jedoch die Einführung weiterer Substanzen in das Gesamtverfahren und die Notwendigkeit, den inhibierten homogenen Katalysator vor dessen erneutem Einsatz wieder zu aktivieren.

Auch EP 0 357 243 A befasst sich mit dem Nachteil der teilweisen Rückspaltung des Addukts aus Ameisensäure und dem tertiären Amin im Verfahren des EP 0 181 078 A durch die gemeinsame Aufarbeitung des Reaktionsaustrags im Verdampfer. Das in EP 0 357 243 A vorgeschlagene Verfahren lehrt bei der katalytischen Hydrierung des Kohlendioxids zu einem Addukt aus Ameisensäure und tertiärem Amin den Einsatz eines homogenen Katalysators enthaltend ein Übergangsmetall der VIII. Nebengruppe (Gruppe 8, 9, 10), eines tertiären Amins und zweier unterschiedlicher Lösungsmittel, nämlich eines unpolaren und eines polaren, jeweils inerten Lösungsmittels, welche zwei nicht-mischbare flüssige Phasen bilden. Als unpolare Lösungsmittel sind aliphatische und aromatische Kohlenwasserstoffe, jedoch auch Phosphine mit aliphatischen und/oder aromatischen Kohlenwasserstoffresten genannt. Als polare Lösungsmittel sind Wasser, Glycerin, Alkohole, Polyole, Sulfolane oder deren Mischungen genannt, wobei Wasser bevorzugt ist. Im unpolaren Lösungsmittel löst sich der homogene Katalysator, im polaren Lösungsmittel das Addukt aus Ameisensäure und tertiärem Amin. Nach Beendigung der Reaktion werden beide Flüssigphasen separiert, beispielsweise durch Dekantierung, und die unpolare Phase enthaltend den homogenen Katalysator und das unpolare Lösungsmittel zur Hydrierstufe zurückgeführt. Die polare Phase enthaltend das Addukt aus Ameisensäure und tertiärem Amin und das polare Lösungsmittel wird dann einem zwingenden Austausch des tertiären Amins im Addukt vor dessen thermischer Spaltung durch eine schwächere, weniger flüchtige Stickstoffbase unterworfen, um die nachfolgende thermische Spaltung zur Darstellung der freien Ameisensäure zu erleichtern beziehungsweise erst möglich zu machen. Analog EP 0 181 078 A sind auch hier Imidazolderivate wie beispielsweise 1-n-Butylimidazol als besonders geeignete schwächere Stickstoffbasen genannt.

Nachteilig am Verfahren des EP 0 357 243 A ist die sehr aufwändige, dreistufige Aufarbeitung des Reaktionsaustrags durch

(i) Trennung der beiden Flüssigphasen und Rückführung der den homogenen Katalysator und das unpolare Lösungsmittel enthaltenden Phase zur Hydrierstufe;

(ii) Austausch des tertiären Amins im Addukt aus Ameisensäure und dem tertiären Amin der anderen Phase durch eine schwächere, weniger flüchtige Stickstoffbase in einem Reaktionskessel mit aufgesetzter Destillationskolonne und Rückführung des freigesetzten tertiären Amins zur Hydrierstufe; und

(iii) thermische Spaltung des Addukts aus Ameisensäure und der schwächeren Stickstoffbase und Rückführung der freigesetzten schwächeren Stickstoffbase zur Basenaustauschstufe.

Ein weiterer Nachteil am Verfahren des EP 0 357 243 A ist der Einsatz zweier Lösungsmittel und somit die Einführung einer weiteren Substanz in das Gesamtverfahren.

Alternativ offenbart EP 0 357 243 A auch die Möglichkeit, nur ein Lösungsmittel einzusetzen. In diesem Fall entfällt die Zugabe des polaren Lösungsmittels, in dem sich ansonsten das Addukt aus Ameisensäure und dem tertiären Amin lösen würde. Das einzige hierbei eingesetzte Lösungsmittel ist das unpolare Lösungsmittel, welches den homogenen Katalysator löst. Auch bei dieser Alternative ist jedoch die sehr aufwändige, dreistufige Aufarbeitung wie oben beschrieben von Nachteil.

DE 44 31 233 A beschreibt ebenfalls die Hydrierung von Kohlendioxid in Gegenwart eines Katalysators enthaltend ein Übergangsmetall der VIII. Nebengruppe (Gruppe 8, 9, 10), eines tertiären Amins und eines polaren Lösungsmittels und Wasser zu einem Addukt aus Ameisensäure und dem tertiären Amin, bei dem jedoch der Katalysator heterogen vorliegt und die Aktivkomponente auf einem inerten Träger aufgebracht ist. Bevorzugte tertiäre Amine sind $C_1$-$C_8$-Trialkylamine, Polyamine mit 2 bis 5 Aminogruppen, aromatische Stickstoffheterocyclen wie Pyridin oder N-Methylimidazol, sowie cyclische und/oder verbrückte Amine wie N-Methylpiperidin, 1,8-Diazabicyclo[5.4.0]undec-7-en oder 1,4-Diazabicyclo[2.2.2]octan. Als geeignete polare Lösungsmittel sind die niedrig siedenden $C_1$-$C_4$-Monoalkohole genannt, wobei analog EP 0 095 321 A sekundäre Alkohole bevorzugt sind. Die Hydrierung erfolgt bei einem Gesamtdruck von 4 bis 20 MPa (40 bis 200 bar) und einer Temperatur von 50 bis 200°C. Zur Aufarbeitung des gebildeten Addukts aus Ameisensäure und tertiärem Amin lehrt DE 44 31 233 A den Einsatz bekannter Verfahren unter explizitem Verweis auf die in EP 0 357 243 A offenbarte Aufarbeitung unter Austausch des tertiären Amins im Addukt aus Ameisensäure und dem tertiären Amin durch eine schwächere, weniger flüchtige Stickstoffbase. Analog dem Verfahren nach EP 0 357 243 A ist auch beim Verfahren nach DE 44 31 233 A die sehr aufwändige, dreistufige Aufarbeitung des Reaktionsaustrags nachteilig.

Aufgabe der vorliegenden Erfindung war es, ein Verfahren zur Herstellung von Ameisensäure durch Hydrierung von Kohlendioxid zur Verfügung zu stellen, das die genannten Nachteile des Standes der Technik nicht oder nur in deutlich vermindertem Ausmaß aufweist und das zu konzentrierter Ameisensäure in hoher Ausbeute und hoher Reinheit führt. Ferner soll das Verfahren eine einfache oder zumindest einfachere Verfahrensführung erlauben, als im Stand der Technik beschrieben, insbesondere ein einfacheres Verfahrenskonzept zur Aufarbeitung des Austrags aus dem Hydrierreaktor, einfachere Verfahrensstufen, eine geringere Anzahl an Verfahrensstufen oder einfachere Apparate. Des Weiteren soll das Verfahren auch mit einem möglichst niedrigen Energiebedarf durchgeführt werden können.

Die Aufarbeitung des Austrags aus dem Hydrierreaktor soll insbesondere ausschließlich mit den im Verfahren bereits befindlichen Stoffen, ohne zusätzliche Hilfsstoffe, erfolgen, und diese sollen im Verfahren vollständig oder weitgehend vollständig recycliert werden.

Die Aufgabe wird gelöst durch ein Verfahren zur Herstellung von Ameisensäure durch Umsetzung von Kohlendioxid mit Wasserstoff in einem Hydrierreaktor in Gegenwart

- eines Katalysators, enthaltend ein Element aus der 8., 9. oder 10. Gruppe des Periodensystems,
- eines tertiären Amins, enthaltend mindestens 12 Kohlenstoffatome pro Molekül, sowie
- eines polaren Lösungsmittels, enthaltend einen oder mehrere Monoalkohole, ausgewählt aus Methanol, Ethanol, Propanolen und Butanolen sowie Wasser,

unter Bildung von Ameisensäure/Amin-Addukten als Intermediaten, die anschließend thermisch gespalten werden, wobei ein tertiäres Amin eingesetzt wird, das einen um mindestens 5 °C höheren Siedepunkt als Ameisensäure aufweist, und wobei

sich in der Hydrierung zwei Flüssigphasen bilden, und zwar eine Unterphase, enthaltend überwiegend das polare Lösungsmittel, in der die Ameisensäure/Amin-Addukte angereichert vorliegen sowie eine Oberphase, enthaltend überwiegend das tertiäre Amin, in der der Katalysator angereichert vorliegt, das dadurch gekennzeichnet ist, dass die Aufarbeitung des Austrags aus dem Hydrierreaktor nach den folgenden Verfahrensschritten durchgeführt wird:

1) Auftrennung der zwei Flüssigphasen aus dem Hydrierreaktor in einem Phasentrenngefäß, Rückführung der Oberphase aus dem Phasentrenngefäß in den Hydrierreaktor und Weiterleitung der Unterphase aus dem Phasentrenngefäß in eine Extraktionsapparatur, worin

2) Reste an Katalysator mit demselben tertiären Amin, das in der Hydrierung eingesetzt wurde, extrahiert und das

mit Katalysator beladene tertiäre Amin in den Hydrierreaktor recycliert wird, und Weiterleitung des katalysatorfreien Stromes von mit den Ameisensäure/Amin-Addukten beladenem polaren Lösungsmittel in eine Destillationseinheit, worin

3) das polare Lösungsmittel als Kopfstrom abgetrennt und in den Hydrierreaktor recycliert wird, unter Erhalt eines Stromes, der

4) in einem Phasentrenngefäß in eine Oberphase enthaltend überwiegend das tertiäre Amin und eine Unterphase, enthaltend überwiegend die Ameisensäure/Amin-Addukte aufgetrennt wird, wobei

5) die Unterphase aus dem Phasentrenngefäß einer thermischen Spalteinheit zugeführt, und darin in einen das tertiäre Amin enthaltenen Strom, der in das Phasentrenngefäß zurückgeführt wird und in reine Ameisensäure gespalten wird, und wobei
ein das tertiäre Amin enthaltende Strom aus dem Phasentrenngefäß in die Extraktionsapparatur als selektives Lösungsmittel für den Katalysator geleitet wird.

Der beim erfindungsgemäßen Verfahren bei der Hydrierung von Kohlendioxid einzusetzende Katalysator ist bevorzugt ein homogener Katalysator. Dieser enthält ein Element aus der 8., 9. oder 10. Gruppe des Periodensystems, also Fe, Co, Ni, Ru, Rh, Pd, Os, Ir und/oder Pt. Bevorzugt enthält der Katalysator Ru, Rh, Pd, Os, Ir und/oder Pt, besonders bevorzugt Ru, Rh und/oder Pd, und ganz besonders bevorzugt Ru.

Die genannten Elemente liegen in Form von komplexartigen Verbindungen im Reaktionsgemisch homogen gelöst vor. Der homogene Katalysator ist dabei so auszuwählen, dass dieser zusammen mit dem tertiären Amin in der Flüssigphase (B) angereichert vorliegt. Unter "angereichert" ist dabei ein Verteilungskoeffizient des homogenen Katalysators

$$P = [\text{Konzentration homogener Katalysator in Flüssigphase (B)}] / [\text{Konzentration homogener Katalysator in Flüssigphase (A)}]$$

von > 1 zu verstehen. Die Auswahl des homogenen Katalysators erfolgt im Allgemeinen durch einen einfachen Versuch, in dem der Verteilungskoeffizient des gewünschten homogenen Katalysators unter den geplanten Verfahrensbedingungen experimentell bestimmt wird.

Flüssigphase (A) ist hierbei die Unterphase aus dem Phasentrenngefäß aus Verfahrensstufe 1).

Wegen ihrer guten Löslichkeit in tertiären Aminen verwendet man beim erfindungsgemäßen Verfahren als homogene Katalysatoren bevorzugt eine metallorganische Komplexverbindung, die ein Element aus der 8., 9. oder 10. Gruppe des Periodensystems und mindestens eine Phosphin-Gruppe mit mindestens einem unverzweigten oder verzweigten, acyclischen oder cyclischen, aliphatischen Rest mit 1 bis 12 Kohlenstoffatomen enthält, wobei einzelne Kohlenstoffatome auch durch >P- substituiert sein können. Im Sinne von verzweigten cyclischen aliphatischen Resten sind damit eingeschlossen auch Reste wie beispielsweise $-CH_2-C_6H_{11}$. Als geeignete Reste sind beispielsweise genannt Methyl, Ethyl, 1-Propyl, 2-Propyl, 1-Butyl, 2-Butyl, 1-(2-Methyl)propyl, 2-(2-Methyl)propyl, 1-Pentyl, 1-Hexyl, 1-Heptyl, 1-Octyl, 1-Nonyl, 1-Decyl, 1-Undecyl, 1-Dodecyl, Cyclopentyl, Cyclohexyl, Cycloheptyl und Cyclooctyl, Methylcyclopentyl, Methylcyclohexyl, 1-(2-Methyl)-pentyl 1-(2-Ethyl)-hexyl, 1-(2-Propyl)heptyl und Norbonyl. Bevorzugt enthält der unverzweigte oder verzweigte, acyclische oder cyclische, aliphatische Rest mindestens 1 sowie bevorzugt maximal 10 Kohlenstoffatome. Im Falle eines ausschließlich cyclischen Restes im oben genannten Sinne beträgt die Zahl der Kohlenstoffatome 3 bis 12 und bevorzugt mindestens 4 sowie bevorzugt maximal 8 Kohlenstoffatome. Bevorzugte Reste sind Ethyl, 1-Butyl, sec-Butyl, 1-Octyl und Cyclohexyl.

Die Phosphin-Gruppe kann einen, zwei oder drei der oben genannten unverzweigten oder verzweigten, acyclischen oder cyclischen, aliphatischen Reste enthalten. Diese können gleich oder verschieden sein. Bevorzugt enthält die Phosphin-Gruppe drei der oben genannten unverzweigten oder verzweigten, acyclischen oder cyclischen, aliphatischen Reste, wobei besonders bevorzugt alle drei Reste gleich sind. Bevorzugte Phosphine sind $P(n\text{-}C_nH_{2n+1})_3$ mit n gleich 1 bis 10, besonders bevorzugt Tri-n-butylphosphin, Tri-n-octylphosphin und 1,2-Bis(dicylohexlphosphino)ethan.

Wie bereits weiter oben erwähnt, können in den genannten unverzweigten oder verzweigten, acyclischen oder cyclischen, aliphatischen Resten einzelne Kohlenstoffatome auch durch >P- substituiert sein. Somit sind auch mehrzähnige, beispielsweise zwei- oder dreizähnige, Phosphin-Liganden mit umfasst. Diese enthalten bevorzugt die Gruppierung $>P-CH_2CH_2-P<$ beziehungsweise

$$>P-CH_2CH_2-\overset{|}{P}-CH_2CH_2-P< .$$

Enthält die Phosphin-Gruppe noch andere Reste als die oben genannten unverzweigten oder verzweigten, acyclischen oder cyclischen, aliphatischen Reste, so entsprechen diese im Allgemeinen denen, die ansonsten üblicherweise bei Phosphin-Liganden für metallorganische Komplexkatalysatoren eingesetzt werden. Als Beispiele seien genannt Phenyl, Tolyl und Xylyl.

Die metallorganische Komplexverbindung kann eine oder mehrere, beispielsweise zwei, drei oder vier, der oben genannten Phosphin-Gruppen mit mindestens einem unverzweigten oder verzweigten, acyclischen oder cyclischen, aliphatischen Rest enthalten. Die restlichen Liganden des metallorganischen Komplexes können verschiedener Natur sein. Als exemplarische Beispiele seien genannt Hydrid, Fluorid, Chlorid, Bromid, Iodid, Formiat, Acetat, Propionat, Carboxylat, Acetylacetonat, Carbonyl, DMSO, Hydroxid, Trialkylamin, Alkoxid.

Die homogenen Katalysatoren können sowohl direkt in ihrer aktiven Form als auch ausgehend von üblichen Standardkomplexen wie beispielsweise $[M(p\text{-}Cymene)Cl_2]_2$, $[M(benzol)Cl_2]_n$, [M(COD)(allyl)], $[MCl_3 \times H_2O]$, $[M(acetylacetonat)_3]$, $[M(DMSO)_4Cl_2]$ mit M gleich Element aus der 8., 9. oder 10. Gruppe des Periodensystems unter Zugabe des beziehungsweise der entsprechenden Phosphin-Liganden erst unter Reaktionsbedingungen erzeugt werden.

Beim erfindungsgemäßen Verfahren bevorzugte homogene Katalysatoren sind $[Ru(P^nBu_3)_4(H)_2]$, $[Ru(P^nOctyl_3)_4(H)_2]$, $[Ru(P^nBu_3)_2$(1,2-bis(dicyclohexylphosphino)-ethan)$(H)_2]$, $[Ru(P^nOctyl_3)_2$(1,2-bis(dicyclohexylphosphino)ethan)$(H)_2]$, $[Ru(PEt_3)_4(H)_2$. Mit diesen können in der Hydrierung von Kohlendioxid TOF-Werte (turn-over-frequency) von größer 1000 $h^{-1}$ erzielt werden.

Beim Einsatz von homogenen Katalysatoren beträgt die eingesetzte Menge der genannten Metallkomponente im metallorganischen Komplex im Allgemeinen 0,1 bis 5000 Gew.-ppm, bevorzugt 1 bis 800 Gew.-ppm und besonders bevorzugt 5 bis 800 Gew.-ppm, jeweils bezogen auf das gesamte flüssige Reaktionsgemisch im Hydrierreaktor.

Der Verteilungskoeffizient des homogenen Katalysators bezogen auf die Menge an Ruthenium in der Aminphase und der das Ameisensäure/Amin-Addukt enthaltenden Produktphase ist nach der Hydrierung im Bereich von P größer 0,5, bevorzugtermaßen größer 1,0 und besonders bevorzugtermaßen größer 4.

Das beim erfindungsgemäßen Verfahren bei der Hydrierung von Kohlendioxid einzusetzende tertiäre Amin weist einen um mindestens 5°C höheren Siedepunkt als Ameisensäure auf. Dabei ist, wie üblich, bei der Angabe der relativen Lage von Siedepunkten von Verbindungen zueinander auf jeweils denselben Druck abzustimmen. Das tertiäre Amin ist dabei so ausgewählt und mit dem polaren Lösungsmittel abgestimmt, dass das tertiäre Amin in der Oberphase im Hydrierreaktor angereichert vorliegt. Unter "angereichert" ist dabei ein Gewichtsanteil von > 50% des freien, das heißt nicht in Form des Ameisensäure/Amin-Addukts gebundenen, tertiären Amins in der Oberphase bezogen auf die Gesamtmenge an freiem, tertiärem Amins in beiden Flüssigphasen zu verstehen. Bevorzugt liegt der Gewichtsanteil bei > 90 %. Die Auswahl des tertiären Amins erfolgt im Allgemeinen durch einen einfachen Versuch, in dem die Löslichkeit des gewünschten tertiären Amins in beiden Flüssigphasen unter den geplanten Verfahrensbedingungen experimentell bestimmt wird. Die Oberphase kann zudem noch Teile des polaren Lösungsmittels sowie eines unpolaren inerten Lösungsmittel enthalten.

Bevorzugt weist das einzusetzende tertiäre Amin einen um mindestens 10°C, besonders bevorzugt einem um mindestens 50°C und ganz besonders bevorzugt einem um mindestens 100°C höheren Siedepunkt als Ameisensäure auf. Eine Einschränkung hinsichtlich eines oberen Grenzwertes für den Siedepunkt ist nicht erforderlich, da für das erfindungsgemäße Verfahren ein möglichst niedriger Dampfdruck des tertiären Amin grundsätzlich von Vorteil ist. Im Allgemeinen liegt der Siedepunkt des tertiären Amins bei einem, gegebenenfalls nach bekannten Methoden vom Vakuum auf 1013 hPa abs hochgerechneten Druck unterhalb von 500°C.

Das beim erfindungsgemäßen Verfahren bevorzugt einzusetzende tertiäre Amin ist ein Amin, enthaltend mindestens 12 Kohlenstoffatome pro Molekül der allgemeinen Formel (Ia)

$$NR^1R^2R^3 \qquad (Ia),$$

in der die Reste $R^1$ bis $R^3$ gleich oder verschieden sind und unabhängig voneinander einen unverzweigten oder verzweigten, acyclischen oder cyclischen, aliphatischen, araliphatischen oder aromatischen Rest mit jeweils 1 bis 16 Kohlenstoffatomen, bevorzugt 1 bis 12 Kohlenstoffatomen, darstellen, wobei einzelne Kohlenstoffatome unabhängig voneinander auch durch eine Heterogruppe ausgewählt aus der Gruppe -O- und >N- substituiert sein können sowie zwei oder alle drei Reste unter Bildung einer mindestens jeweils vier Atome umfassenden Kette auch miteinander verbunden sein können.

Als geeignete Amine seien beispielsweise genannt:

- Tri-n-butylamin, Tri-n-pentylamin, Tri-n-hexylamin, Tri-n-heptylamin, Tri-n-octylamin, Tri-n-nonylamin, Tri-n-decylamin, Tri-n-undecylamin, Tri-n-dodecylamin, Tri-n-tridecylamin, Tri-n-tetradecylamin, Tri-n-pentadecylamin, Tri-n-hexadecylamin, Tri-(2-ethylhexyl)amin.

- Dimethyl-decylamin, Dimethyl-dodecylamin, Dimethyl-tetradecylamin, Ethyl-di-(2-propyl)amin ($Sdp_{1013\ hPa}$ = 127°C), Dioctyl-methylamin, Dihexyl-methylamin.
- Tricyclopentylamin, Tricyclohexylamin, Tricycloheptylamin, Tricyclooctylamin und deren durch eine oder mehrere Methyl-, Ethyl-, 1-Propy-, 2-Propyl-, 1-Butyl-, 2-Butyl- oder 2-Methyl-2-propyl-Gruppen substituierte Derivate.
- Dimethyl-cyclohexylamin, Methyl-dicyclohexylamin, Diethyl-cyclohexylamin, Ethyl-dicyclohexylamin, Dimethyl-cyclopentylamin, Methyl-dicyclopentylamin.
- Triphenylamin, Methyl-diphenylamin, Ethyl-diphenylamin, Propyl-diphenylamin, Butyl-dipehnylamin, 2-Ethyl-hexyl-diphenylamin, Dimethyl-phenylamin, Diethylphenylamin, Dipropyl-phenylamin, Dibutyl-phenylamin, Bis(2-Ethyl-hexyl)-phenylamin, Tribenzylamin, Methyl-dibenzylamin, Ethyl-dibenzylamin und deren durch eine oder mehrere Methyl-, Ethyl-, 1-Propy-, 2-Propyl-, 1-Butyl-, 2-Butyl- oder 2-Methyl-2-propyl-Gruppen substituierte Derivate.
- N-$C_1$-bis-$C_{12}$-Alkyl-piperidine, N,N-Di-$C_1$-bis-$C_{12}$-Alkyl-piperazine, N-$C_1$-bis-$C_{12}$-Alkyl-pyrrolidine, N-$C_1$-bis-$C_{12}$-Alkyl-imidazole und deren durch eine oder mehrere Methyl-, Ethyl-, 1-Propy-, 2-Propyl-, 1-Butyl-, 2-Butyl- oder 2-Methyl-2-propyl-Gruppen substituierte Derivate.
- 1,8-Diazabicyclo[5.4.0]undec-7-en ("DBU"), 1,4-Diazabicyclo[2.2.2]octan ("DABCO"), N-Methyl-8-azabicyclo[3.2.1]octan ("Tropan"), N-Methyl-9-azabicyclo[3.3.1]nonan ("Granatan"), 1-Azabicyclo[2.2.2]octan ("Chinuclidin").

Beim erfindungsgemäßen Verfahren können natürlich auch Gemische einer beliebigen Anzahl verschiedener tertiärer Amine eingesetzt werden.

Besonders bevorzugt setzt man beim erfindungsgemäßen Verfahren als tertiäres Amin ein Amin der allgemeinen Formel (la), in der die Reste $R^1$ bis $R^3$ unabhängig voneinander ausgewählt sind aus der Gruppe $C_1$- bis $C_{12}$-Alkyl, $C_5$- bis $C_8$-Cycloalkyl, Benzyl und Phenyl, ein.

Besonders bevorzugt setzt man beim erfindungsgemäßen Verfahren als tertiäres Amin ein gesättigtes Amin, d.h. nur Einfachbindungen enthaltend, der allgemeinen Formel (la) ein.

Ganz besonders bevorzugt setzt man beim erfindungsgemäßen Verfahren als tertiäres Amin ein Amin der allgemeinen Formel (la) ein, in der die Reste $R^1$ bis $R^3$ unabhängig voneinander ausgewählt sind aus der Gruppe $C_5$- bis $C_8$-Alkyl, insbesondere Tri-n-pentylamin, Tri-n-hexylamin, Tri-n-heptylamin, Tri-n-octylamin, Dimethylcyclohexylamin, Methyldicyclohexylamin, Dioctyl-methylamin und Dimethyldecylamin.

Insbesondere setzt man als tertiäres Amin ein Amin der allgemeinen Formel (Ia) ein, in der die Reste $R^1$ bis $R^3$ unabhängig voneinander ausgewählt sind aus $C_5$- und $C_6$-Alkyl.

Bevorzugt liegt das tertiäre Amin beim erfindungsgemäßen Verfahren in allen Verfahrensstufen flüssig vor. Dies ist jedoch kein zwingendes Erfordernis. Es würde auch ausreichen, wenn das tertiäre Amin zumindest in geeigneten Lösungsmitteln gelöst wäre. Geeignete Lösungsmittel sind prinzipiell solche, welche hinsichtlich der Hydrierung von Kohlendioxid sowie der thermischen Spaltung des Addukts chemisch inert sind, in denen sich das tertiäre Amin und, im Falle des Einsatzes eines homogenen Katalysators, auch diesen gut lösen, umgekehrt polares Lösungsmittel sowie das Ameisensäure/Amin-Addukte schlecht lösen. In Frage kommen daher prinzipiell chemisch inerte, unpolare Lösungsmittel wie etwa aliphatische, aromatische oder araliphatische Kohlenwasserstoffe, beispielsweise Octan und höhere Alkane, Toluol, Xylole.

Das beim erfindungsgemäßen Verfahren bei der Hydrierung von Kohlendioxid einzusetzende polare Lösungsmittel weist einen um mindestens 5°C niedrigeren Siedepunkt als die zur Spaltung der Ameisensäure/Amin-Addukte bei gleichem Druck benötigte Temperatur auf. Das polare Lösungsmittel ist dabei so auszuwählen beziehungsweise mit dem tertiären Amin abzustimmen, dass das polare Lösungsmittel in der Unterphase angereichert vorliegt. Unter "angereichert" ist dabei ein Gewichtsanteil von > 50% des polaren Lösungsmittels in der Unterphase bezogen auf die Gesamtmenge an polarem Lösungsmittel in beiden Flüssigphasen zu verstehen. Bevorzugt liegt der Gewichtsanteil bei > 70%. Die Auswahl des polaren Lösungsmittels erfolgt im Allgemeinen durch einen einfachen Versuch, in dem die Löslichkeit des gewünschten polaren Lösungsmittels in beiden Flüssigphasen unter den geplanten Verfahrensbedingungen experimentell bestimmt wird.

Bei dem polaren Lösungsmittel kann es sich um ein reines polares Lösungsmittel als auch um ein Gemisch verschiedener polarer Lösungsmittel handeln, solange die an das Lösungsmittel gestellten oben genannten Bedingungen bezüglich, Siedepunkt und Phasenverhalten zutreffen.

Bevorzugt weist das einzusetzende polare Lösungsmittel einen um mindestens 10°C, besonders bevorzugt einen um mindestens 50°C niedrigeren Siedepunkt als die zur Spaltung der Ameisensäure/Amin-Addukte bei gleichem Druck benötigte Temperatur auf. Bei Lösungsmittelgemischen liegen die Siedpunkte des eingesetzten Lösungsmittelgemisches bzw. eines Azeotrops oder Heteroazeotrops um mindestens 10°C, besonders bevorzugt einem um mindestens 50°C niedriger als die zur Spaltung der Ameisensäure/Amin-Addukte bei gleichem Druck benötigte Temperatur.

Als Stoffklassen, die als polare Lösungsmittel geeignet sind, kommen bevorzugt Alkohole sowie deren Ameisensäureester und Wasser in Frage. Die Alkohole weisen einen um mindestens 10°C, besonders bevorzugt einem um mindestens 50°C niedrigeren Siedepunkt als die zur Spaltung der Ameisensäure/Amin-Addukte bei gleichem Druck benötigte Temperatur auf, um die Veresterung der Alkohole mit Ameisensäure möglichst gering zu halten.

Als geeignete Alkohole sind Alkohole genannt, in welchen sich im Gemisch mit Wasser die Trialkylammoniumformiate bevorzugt lösen und diese Produktphase eine Mischungslücke mit dem freien Trialkylamin aufweist. Als geeignete Alkohole seien Beispielsweise Methanol, Ethanol, 2-Methoxyethanol, 1-Propanol, 2-Propanol, 1-Butanol, 2-Butanol, 2-Methyl-1-propanol genannt. Das Verhältnis von Alkohol zu Wasser ist so zu wählen, das sich zusammen mit dem Trialkylammoniumformiates und dem Trialkylamin ein zweiphasiges Gemisch bildet, in welchem sich der Großteil des Trialkylammoniumformiates, das Wasser sowie das polare Lösungsmittel in der Unterphase befindet, was im Allgemeinen durch einen einfachen Versuch bestimmt wird, in dem die Löslichkeit des gewünschten polaren Lösungsmittelgemisches in beiden Flüssigphasen unter den geplanten Verfahrensbedingungen experimentell bestimmt wird.

Das Molverhältnis des beim erfindungsgemäßen Verfahren einzusetzenden polaren Lösungsmittels oder Lösungsmittelgemisches zum eingesetzten tertiären Amin beträgt im Allgemeinen 0,5 bis 30 und bevorzugt 1 bis 20.

Das bei der Hydrierung von Kohlendioxid einzusetzende Kohlendioxid kann fest, flüssig oder gasförmig eingesetzt werden. Es ist auch möglich, großtechnisch verfügbare, Kohlendioxid enthaltende Gasgemische zu verwenden, sofern diese weitgehend frei von Kohlenmonoxid (Volumenanteil von <1 % CO) sind. Der bei der Hydrierung von Kohlendioxid einzusetzende Wasserstoff ist im Allgemeinen gasförmig. Kohlendioxid und Wasserstoff können auch inerte Gase, wie etwa Stickstoff oder Edelgase, enthalten. Vorteilhafterweise liegt jedoch deren Gehalt unterhalb von 10 mol% bezogen auf die Gesamtmenge an Kohlendioxid und Wasserstoff im Hydrierreaktor. Größere Mengen sind zwar gegebenenfalls ebenfalls noch tolerierbar, bedingen aber im Allgemeinen die Anwendung eines höheren Druckes im Reaktor, wodurch weitere Kompressionsenergie erforderlich ist.

Die Hydrierung von Kohlendioxid erfolgt in der Flüssigphase bevorzugt bei einer Temperatur von 20 bis 200°C und einem Gesamtdruck von 0,2 bis 30 MPa abs. Bevorzugt beträgt die Temperatur mindestens 30°C und besonders bevorzugt mindestens 40°C sowie bevorzugt höchstens 150°C, besonders bevorzugt höchstens 120°C und ganz besonders bevorzugt höchstens 100°C. Der Gesamtdruck beträgt bevorzugt mindestens 1 MPa abs und besonders bevorzugt mindestens 5 MPa abs sowie bevorzugt höchstens 30 MPa abs.

Der Partialdruck des Kohlendioxids beträgt dabei im Allgemeinen mindestens 0,5 MPa und bevorzugt mindestens 2 MPa sowie im Allgemeinen höchstens 8 MPa. Der Partialdruck des Wasserstoffs beträgt im Allgemeinen mindestens 0,5 MPa und bevorzugt mindestens 1 MPa sowie im Allgemeinen höchstens 25 MPa und bevorzugt höchstens 10 MPa.

Das Molverhältnis von Wasserstoff zu Kohlendioxid im Zulauf des Hydrierreaktors beträgt bevorzugt 0,1 bis 10 und besonders bevorzugt 1 bis 3.

Das Molverhältnis von Kohlendioxid zu tertiärem Amin im Zulauf des Hydrierreaktors beträgt im Allgemeinen 0,1 bis 10 und bevorzugt 0,5 bis 3.

Als Hydrierreaktoren können prinzipiell alle Reaktoren eingesetzt werden, welche für gas/flüssig-Reaktionen unter der gegebenen Temperatur und dem gegebenen Drucks grundsätzlich geeignet sind. Geeignete Standardreaktoren für flüssig-flüssig Reaktionssysteme sind beispielsweise in K.D. Henkel, "Reactor Types and Their Industrial Applications", in Ullmann's Encyclopedia of Industrial Chemistry, 2005, Wiley-VCH Verlag GmbH & Co. KGaA, DOI: 10.1002/14356007.b04_087, Kapitel 3.3 "Reactors for gas-liquid reactions" angegeben. Als Beispiele seien genannt Rührkesselreaktoren, Rohrreaktoren oder Blasensäulenreaktoren.

Die Hydrierung von Kohlendioxid kann beim erfindungsgemäßen Verfahren diskontinuierlich oder kontinuierlich durchgeführt werden. Bei der diskontinuierlichen Fahrweise wird der Reaktor mit den gewünschten flüssigen und gegebenenfalls festen Einsatz- und Hilfsstoffen bestückt und anschließend Kohlendioxid und Wasserstoff auf den gewünschten Druck bei der gewünschten Temperatur aufgepresst. Nach Ende der Reaktion wird der Reaktor im Regelfall entspannt und die beiden gebildeten Flüssigphasen voneinander getrennt. Bei der kontinuierlichen Fahrweise werden die Einsatz- und Hilfsstoffe, einschließlich des Kohlendioxids und Wasserstoffs kontinuierlich zugegeben. Entsprechend wird die Flüssigphase kontinuierlich aus dem Reaktor abgeführt, so dass der Flüssigkeitsstand im Reaktor im Mittel gleich bleibt. Bevorzugt ist die kontinuierliche Hydrierung von Kohlendioxid.

Die mittlere Verweilzeit im Hydrierreaktor beträgt im Allgemeinen 10 Minuten bis 5 Stunden.

Die bei der Hydrierung von Kohlendioxid in Gegenwart des einzusetzenden Katalysators, des polaren Lösungsmittels und des tertiären Amins gebildeten Ameisensäure/Amin-Addukte weisen im Allgemeinen die allgemeine Formel (IIa)

$$NR^1R^2R^3 \cdot x_i\ HCOOH \qquad (IIa),$$

auf, in der die Reste $R^1$ bis $R^3$ den beim tertiären Amin (Ia) beschriebenen Resten entsprechen und $x_1$ gleich 0.4 bis 5, bevorzugt 0.7 bis 1.6 beträgt. Die jeweiligen gemittelten Zusammensetzungen der Amin-Ameisensäureverhältnisse in den Produktphasen bei den jeweiligen Verfahrensschritten, d.h. der Faktor $x_i$, lassen sich beispielsweise durch Bestimmung des Ameisensäuregehaltes durch Titration mit einer alkoholischen KOH-Lösung gegen Phenolphthalein und des Amingehaltes durch Gaschromatographie bestimmen. Die Zusammensetzung der Ameisensäure/Amin-Addukte, d.h. der Faktor $x_i$ kann sich während der verschiedenen Verfahrensschritte ändern. So bilden sich zum Beispiel im Allgemeinen nach der Entfernung des polaren Lösungsmittel Addukte mit einem höheren Ameisensäuregehalt mit $x_2 > x_1$ mit $x_2$ 1 bis 4 aus, wobei das überschüssige, freie Amin eine Zweitphase ausbilden kann.

Bei der Hydrierung von Kohlendioxid nach dem erfindungsgemäßen Verfahren werden zwei Flüssigphasen gebildet. Die Unterphase ist mit den Ameisensäure/Amin-Addukten sowie dem polaren Lösungsmittel angereichert. Hinsichtlich der Ameisensäure/Amin-Addukte ist unter "angereichert" ein Verteilungskoeffizient der Ameisensäure/Amin-Addukte

$$P = [\text{Konzentration Ameisensäure/Amin-Addukt (II) in Flüssigphase (A)}] / [\text{Konzentration Ameisensäure/Amin-Addukt (II) in Flüssigphase (B)}]$$

von > 1 zu verstehen. Bevorzugt liegt der Verteilungskoeffizient bei ≥ 2 und besonders bevorzugt bei ≥ 5. Die Oberphase ist mit dem tertiären Amin angereichert. Im Falle des Einsatzes eines homogenen Katalysators liegt dieser ebenfalls in der Oberphase angereichert vor.

Die Flüssigphasen (A) und (B) haben dabei die bereits vorstehend definierte Bedeutung.

Die beiden gebildeten Flüssigphasen werden beim erfindungsgemäßen Verfahren voneinander getrennt und die Oberphase zum Hydrierreaktor rückgeführt. Gegebenenfalls ist auch eine Rückführung einer über beiden Flüssigphasen vorhandenen weiteren Flüssigphase enthaltend nicht umgesetztes Kohlendioxid sowie einer Gasphase enthaltend nicht umgesetztes Kohlendioxid und/oder nicht umgesetzten Wasserstoff zum Hydrierreaktor vorteilhaft. Gegebenenfalls ist es beispielsweise zur Ausschleusung von unerwünschten Nebenprodukten oder Verunreinigungen wünschenswert, einen Teil der Oberphase und/oder einen Teil der Kohlendioxid oder Kohlendioxid und Wasserstoff enthaltenden flüssigen beziehungsweise gasförmigen Phasen aus dem Verfahren auszuschleusen.

Die Trennung der beiden Flüssigphasen erfolgt im Allgemeinen durch gravimetrische Phasentrennung. Als Phasentrenngefäße sind beispielsweise Standardapparaturen und Standardmethoden geeignet, welche sich zum Beispiel in E. Müller et al., "Liquid-Liquid Extraction", in Ullmann's Encylcopedia of Industrial Chemistry, 2005, Wiley-VCH Verlag GmbH & Co. KGaA, DOI:10.1002/14356007.b03_06, Kapitel 3 "Apparatus" finden lassen. Im Allgemeinen ist die mit den Ameisensäure/Amin-Addukten sowie dem polaren Lösungsmittel angereicherte Flüssigphase schwerer und bildet die Unterphase.

Die Phasentrennung kann beispielsweise nach Entspannung, beispielsweise auf etwa oder nahe Atmosphärendruck, und Abkühlung des flüssigen Reaktionsgemischs, beispielsweise auf etwa oder nahe Umgebungstemperatur, erfolgen. Allerdings ist dabei zu befürchten, dass zumindest ein Teil des bei dem höherem Reaktionsdruck in den Flüssigphasen gelösten Gases, insbesondere Kohlendioxid, bei der Entspannung entgast und als Gasstrom separat zu verdichten und zum Hydrierreaktor rückzuführen ist. Ebenso ist auch die Unterphase vor der Rückführung zum Hydrierreaktor separat auf Reaktionsdruck zu bringen. Dabei ist für die rückzuführende Gas- und Flüssigphase jeweils ein geeigneter, entsprechend der zu überwindenden Druckdifferenz ausgelegter Verdichter bzw. eine Pumpe vorzusehen, welche im Betrieb zudem zusätzliche Energie verbraucht.

Im Rahmen der vorliegenden Erfindung wurde gefunden, dass sich bei dem vorliegenden System, das heißt, einer mit den Ameisensäure/Amin-Addukten sowie dem polaren Lösungsmittel angereicherten Unterphase und einer mit dem tertiären Amin und im Falle des Einsatzes eines homogenen Katalysators auch mit diesem angereicherten Oberphase, beide Flüssigphasen sich auch bei einem deutlich erhöhten Druck sehr gut voneinander trennen können. Daher ist beim erfindungsgemäßen Verfahren das Lösungsmittel bevorzugt so auszuwählen, dass die Trennung der einen, mit den Ameisensäure/Amin-Addukten und dem polaren Lösungsmittel angereicherte Unterphase von der anderen, mit dem tertiären Amin angereicherten Oberphase, sowie die Rückführung der Oberphase zum Hydrierreaktor bei einem Druck von 1 bis 30 MPa abs durchgeführt werden kann. Gemäß dem Gesamtdruck im Hydrierreaktor, liegt der Druck bei bevorzugt höchstens 30 MPa abs. So kann es sogar möglich sein, ohne vorhergehende Entspannung beide Flüssigphasen voneinander zu trennen und die Oberphase ohne nennenswerte Druckerhöhung zum Hydrierreaktor zurückführen zu können. In diesem Falle, sowie auch im Falle einer nur geringfügigen Entspannung ist es möglich, auf eine Rückführung einer etwaigen Gasphase gänzlich zu verzichten. Ob dieser Verzicht für das jeweils konkrete System möglich ist, ist im Zweifel vorab durch einfache experimentelle Beispiele zu ermitteln.

Das erfindungsgemäße Verfahren kann somit bevorzugt in der Weise durchgeführt werden, dass der Druck und die Temperatur im Hydrierreaktor und im Phasentrenngefäß gleich oder annähernd gleich sind, wobei unter annähernd gleich vorliegend ein Druckunterschied von bis zu +/- 5 bar bzw. ein Temperaturunterschied von bis zu +/-5°C verstanden wird.

In einer Ausführungsform erfolgt die Phasentrennung bei einem Druck von mindestens 50%, ganz besonders bevorzugt von mindestens 90% und insbesondere von mindestens 95% des Reaktionsdrucks. Der Druck bei der Phasentrennung liegt besonders bevorzugt bei höchstens 105% und ganz besonders bevorzugt bei höchstens 100% des Reaktionsdrucks.

Überraschenderweise wurde auch gefunden, dass sich bei dem vorliegendem System beide Flüssigphasen auch bei einer erhöhten Temperatur, die der Reaktionstemperatur entspricht, sehr gut voneinander trennen. Insofern ist zur Phasentrennung auch keine Abkühlung und anschließende Aufheizung der rückzuführenden Oberphase erforderlich, was ebenfalls Energie einspart.

Der Großteil des polaren Lösungsmittels der abgetrennten Unterphase wird in einer Destillationseinheit thermisch von den Ameisensäure/Amin-Addukten abgetrennt, wobei das destillativ entfernte polare Lösungsmittel zum Hydrierreaktor rückgeführt wird. Im Sumpf der Destillationseinheit fallen die reinen Ameisensäure/Amin-Addukte sowie freies Amin an, da sich bei der Entfernung des polaren Lösungsmittels Ameisensäure/Amin-Addukte mit niedrigerem Amingehalt bilden, wodurch sich ein zweiphasiges Sumpfgemisch bestehend aus einer Amin- und einer Ameisensäure/Amin-Addukt-Phase bilden kann.

Die thermische Trennung des polaren Lösungsmittels oder -gemisches, siehe oben, erfolgt bevorzugtermaßen bei einer Sumpftemperatur, bei welcher bei gegebenem Druck sich keine freie Ameisensäure aus dem Ameisensäure/Amin-Addukt mit dem höheren (x1) oder niedriegerem (x2) Amingehalt bildet. Im Allgemeinen beträgt die Sumpftemperatur der thermischen Abtrenneinheit mindestens 20°C, bevorzugtermaßen mindestens 50°C und besonders bevorzugtermaßen mindestens 70°C sowie im Allgemeinen höchstens 210°C, bevorzugtermaßen höchstens 190°C. Der Druck beträgt dabei im Allgemeinen mindestens 1 hPa abs, bevorzugt mindestens 50 hPa abs und besonders bevorzugt mindestens 100 hPa abs sowie im Allgemeinen höchstens 1 MPa abs und bevorzugtermaßen 0,1 MPa abs.

Die thermische Abtrennung des polaren Lösungsmittels oder -gemisches erfolgt entweder in einem Verdampfer oder in einer Destillationseinheit, bestehend aus Verdampfer und Kolonne, gefüllt mit Packung, Füllkörpern und/oder Böden. Das Lösungsmittel kann nach der thermischen Trennung kondensiert werden, wobei wiederum die dabei freiwerdende Kondensationsenthalpie genutzt werden kann, um beispielsweise das aus der Extraktion kommende Lösungsmittel mit Amin/Ameisensäureaddukt-Gemisch auf Abdampftemperatur vorzuwärmen.

Alternativ können auch nur Teile des Lösungsmittelgemisches abgetrennt werden. Dies gilt insbesondere für Lösungsmittelkomponenten, die in der späteren Ameisensäuredestillation über einen Seitenstrom abgetrennt werden können. (Stichwort wässrige Ameisensäure).

Die Ameisensäure/Amin-Addukte, die nach der thermischen Abtrennung des polaren Lösungsmittels oder -gemisches oder Teilen des Lösungsmittels anfallen, werden dann in einer Destillationseinheit thermisch in freie Ameisensäure und freies tertiäres Amin gespalteten, wobei die gebildete freie Ameisensäure destillativ entfernt und das im Sumpf der Destillationseinheit enthaltene freie tertiäre Amin zum Hydrierreaktor rückführt wird. Dabei kann das bei der thermischen Abtrennung des polaren Lösungsmittels als Zweitphase anfallende freie Amin zuvor in einem Phasentrenngefäß abgetrennt werden, in einem gemeinsamen Phasentrenngefäß zusammen mit dem Sumpfprodukt der thermischen Spalteinheit zur Ameisensäureabtrennung oder als zweiphasiges Gemisch direkt der Spalteinheit zugeführt werden (siehe allgemeine Ausführungsformen). Die Entnahme der freigesetzten Ameisensäure kann dabei beispielsweise (i) über Kopf, (ii) über Kopf und als Seitenabzug oder (iii) nur als Seitenabzug erfolgen. Wird Ameisensäure über Kopf entnommen, so ist dabei eine Ameisensäure-Reinheit von bis zu 99,99 Gew.-% möglich. Bei einer Entnahme als Seitenabzug wird wässrige Ameisensäure erhalten, wobei hierbei das Gemisch mit etwa 85 Gew.-% Ameisensäure in der Praxis von besonderer Bedeutung ist. Je nach dem Wassergehalt des Zulaufs zur Destillationseinheit wird die Ameisensäure verstärkt als Kopfprodukt oder als Seitenprodukt entnommen. Bei Bedarf ist es sogar möglich, Ameisensäure nur als Seitenprodukt zu entnehmen, wobei dann gegebenenfalls die erforderliche Wassermenge sogar noch explizit zugegeben werden kann. Die thermische Spaltung des Ameisensäure/Amin-Addukts erfolgt im Allgemeinen nach den im Stand der Technik bekannten Verfahrensparametern hinsichtlich Druck, Temperatur sowie apparativer Ausgestaltung. So sei beispielsweise auf die Beschreibungen in EP 0 181 078 A oder WO 2006/021,411 verwiesen. Die einzusetzende Destillationseinheit umfasst in der Regel eine Destillationskolonne, welche im Allgemeinen Füllkörper, Packungen und/oder Böden enthält.

Im Allgemeinen beträgt die Sumpftemperatur in der Destillationskolonne mindestens 130°C, bevorzugt mindestens 140°C und besonders bevorzugt mindestens 150°C, sowie im Allgemeinen höchstens 210°C, bevorzugt höchstens 190°C und besonders bevorzugt höchstens 185°C. Der Druck beträgt im Allgemeinen mindestens 1 hPa abs, bevorzugt mindestens 50 hPa abs und besonders bevorzugt mindestens 100 hPa abs, sowie im Allgemeinen höchstens 500 hPa abs, bevorzugt höchstens 300 hPa abs und besonders bevorzugt höchstens 250 hPa abs.

Gegebenenfalls wird als Seitenprodukt noch ein wasserhaltiger Strom an Ameisensäure entnommen. Bei einem

Zusatz von Wasser, beispielsweise zur Förderung der Hydrierung, ist dies sogar besonders vorteilhaft.

Die Lösung des Addukts aus tertiärem Amin und Ameisensäure wird mit aus den entsprechenden Phasentrenngefäßen stammendem Strömen von freiem tertiärem Amin extrahiert und in den Hydrierreaktor rückgeführt. Dies geschieht, um Restmengen an Hydrierkatalysator aus dem Produktstrom abzutrennen. Ohne diese Extraktion könnte Hydrierkatalysator in die Vorrichtung zur thermischen Spaltung des Addukts aus tertiärem Amin und Ameisensäure gelangen, die Zersetzung von Ameisensäure katalysieren und so die Ameisensäureausbeute mindern. Restmengen aus Wasserstoff und Kohlendioxid werden als Abgas entsorgt.

Die Extraktion erfolgt bei Temperaturen von 30 bis 100 °C und Drücken von 1 bis 80 bar. Die Extraktion kann auch unter Wasserstoffdruck durchgeführt werden.

Die Extraktion des Hydrierkatalysators kann in jeder geeigneten, dem Fachmann bekannten Vorrichtung durchgeführt werden, bevorzugt in Gegenstrom-Extraktionskolonnen, Mixer-Settler-Kaskaden oder Kombinationen von Mixer-Settlern mit Kolonnen.

Der Produktstrom aus der Extraktionsvorrichtung, der aus einer Lösung des Ameisensäure/Amin-Addukts in dem jeweiligen Lösungsmittel oder Lösungsmittelgemisch besteht, wird in die thermische Spalteinheit geleitet, um das polare Lösungsmittel oder - gemisch vom Ameisensäure/Amin-Addukt zu trennen. Die aus der Extraktionsvorrichtung tertiäres Amin und Hydrierkatalysator enthaltende Phase wird in die Hydrierstufe zurückgeführt.

Unter Umständen werden neben dem Katalysator Anteile einzelner Komponenten des polaren Lösungsmittels aus der zu extrahierenden Flüssigphase im Extraktionsmittel, dem Aminstrom, gelöst. Dies ist für das Verfahren kein Nachteil, da die bereits extrahierte Menge an Lösungsmittel nicht der Lösungsmittelabtrennung zugeführt werden muss und somit unter Umständen Verdampfungsenergie einspart.

Es kann vorteilhaft sein, zwischen der Extraktionsapparatur und der thermischen Trennvorrichtung eine Vorrichtung zur Adsorption von Spuren von Hydrierkatalysator zu integrieren. Zahlreiche Adsorptionsmittel sind für die Adsorption geeignet. Beispiele hierfür sind Polyacrylsäure und Salze dieser, sulfonierte Polystyrole und Salze dieser, Aktivkohlen, Montmorillonite, Bentonite, Kieselgele sowie Zeolithe.

Falls die Menge an Hydrierkatalysator im Produktstrom des Phasentrenngefäßes B kleiner als 1 ppm, insbesondere kleiner als 0.1 ppm ist, reicht die Adsorptionsvorrichtung für die Abtrennung des Hydrierkatalysators und seine Rückgewinnung aus. Dann kann die Extraktionsstufe entfallen und das tertiäre Amin zusammen mit dem organischen Lösungsmittel in die Hydrierstufe zurückgeführt werden.

Das erfindungsgemäße Verfahren besitzt gegenüber den in EP 181.078 B1 und EP 357.243 B1 vorbeschriebenen integrierten Verfahren eine Reihe von Vorteilen: Für das Abfangen der Ameisensäure in der Hydrierung und die thermische Spaltung der Ameisensäure/Amin-Addukte wird das gleiche tertiäre Amin verwendet. Dieses Amin, das bei der thermischen Spaltung frei anfällt, wird dann zur Extraktion von Katalysatorresten in der Produktphase eingesetzt werden, um Spuren des Katalysators mit dem Amin in das Reaktionsgefäß zurückzuführen. Es besitzt eine höhere Stabilität als die vorbeschriebenen N-Alkylimidazole. Verluste an Edelmetallen treten praktisch nicht auf. Es wird verhindert, dass der Katalysator in die thermische Spalteinheit gelangt und darin die Zersetzung von Ameisensäure katalysiert. Von großem Vorteil ist, dass er in seiner aktiven Form abgetrennt und zurückgeführt werden kann. Es werden hohe Ameisensäure-Ausbeuten und eine hohe Produktreinheit erzielt. An die Stelle von zwei Destillationen treten Extraktionen und Phasentrennungen. Hierdurch werden Energie- und Investitionskosten gesenkt. Auch kann die erste Phasentrennung unter Druck durchgeführt werden, was niedrigere Abgasströme zur Folge hat.

Die Erfindung wird im Folgenden anhand von Ausführungsbeispielen sowie einer Zeichnung näher erläutert.

Beispiele

Beispiele A-1 bis A-17 (Hydrierung und Phasentrennung, d.h Verfahrensschritt 1 der Aufarbeitung des Austrages aus dem Hydrierreaktor (I))

Ein 250 mL Autoklav aus Hastelloy C, ausgestattet mit einem Magnetrührstab, wurde unter Inertbedingungen mit tertiärem Amin, polarem Lösungsmittel und homogenem Katalysator befüllt. Anschließend wurde der Autoklav verschlossen und bei Raumtemperatur $CO_2$ aufgepresst. Nachfolgend wurde $H_2$ aufgepresst und der Reaktor unter Rühren (700 U/min) erwärmt. Nach der gewünschten Reaktionszeit wurde der Autoklav abgekühlt und das Reaktionsgemisch entspannt. Es wurde - sofern nicht anders vermerkt - ein zweiphasiges Produktgemisch erhalten, wobei die obere Phase mit dem noch freien tertiären Amin und dem homogenen Katalysator, und die untere Phase mit dem polaren Lösungsmittel und dem gebildeten Ameisensäure/Amin-Addukt angereichert war. Der Gesamtgehalt an Ameisensäure im Ameisensäure/Amin-Addukt wurde durch Titration mit 0,1 N KOH in MeOH potentiometrisch mit einem "Mettler Toledo DL50"-Titrator bestimmt. Daraus wurden die Turnover Frequency (=TOF; zur Definition der TOF siehe: J.F. Hartwig, Organotransition Metal Chemistry, 1. Auflage, 2010, Universtiy Science Books, Sausalito/California S.545) und die Reaktionsrate berechnet. Die Zusammensetzung der beiden Phasen wurde durch Gaschromatographie bestimmt. Der Rutheniumgehalt wurde per Atomadsorptionsspektroskopie (=AAS) bestimmt. Die Parameter und Ergebnisse der einzelnen

Versuche sind in Tabelle 1.1 bis 1.5 wiedergegeben.

Die Beispiele A-1 bis A-17 zeigen, dass beim erfindungsgemäßen Verfahren selbst unter Variation des tertiären Amins, des polaren Lösungsmittels, des Katalysators hinsichtlich der Liganden und der Metallkomponente, der Menge des Katalysators, sowie der zugegebenen Wassermenge hohe bis sehr hohe Reaktionsraten von bis zu 0,98 mol $kg^{-1}$ $h^{-1}$ erzielt werden. Alle untersuchten Systeme bildeten zwei Phasen, wobei die obere Phase jeweils mit dem noch freien tertiären Amin und dem homogenen Katalysator, und die untere Phase jeweils mit dem polaren Lösungsmittel und dem gebildeten Ameisensäure/Amin-Addukt angereichert war.

**Tabelle 1.1**

| | Beispiel A-1 | Beispiel A-2 | Beispiel A-3 | Beispiel A-4 |
|---|---|---|---|---|
| Tertiäres Amin | 75 g Trihexylamin | 75 g Trihexylamin | 75 g Tripentylamin | 75 g Tripentylamin |
| Polares Lösungsmittel (eingesetzt) | 17.8 g 1-Propanol<br>7.3 g Wasser | 21.7 g 2-Propanol<br>3.3 g Wasser | 17.8 g 1-Propanol<br>7.3 g Wasser | 17.8 g 1-Propanol<br>7.3 g Wasser |
| Katalysator | 0.2 g<br>$[Ru(P^nBu_3)_4(H)_2]$ | 0.2 g<br>$[Ru(P^nBu_3)_4(H)_2]$ | 0.3 g<br>$[Ru(P^nOct_3)_4(H)_2]$ | 0.2 g<br>$[Ru(P^nBu_3)_4(H)_2]$ |
| Aufpressen von $CO_2$ | Mit 19.6 g auf 2.4 MPa abs | Mit 20.0 g auf 2.3 MPa abs | Mit 20.0 g auf 2.5 MPa abs | Mit 20.3 g auf 2.5 MPa abs |
| Aufpressen von $H_2$ | Auf 10.4 MPa abs | Auf 10.3 MPa abs | Auf 10.6 MPa abs | Auf 10.5 MPa abs |
| Erwärmen | Auf 50°C | Auf 50°C | Auf 50°C | Auf 50°C |
| Druckänderung | Auf 10.0 MPa abs | Auf 10.5 MPa abs | Auf 11.4 MPa abs | Auf 11.5 MPa abs |
| Reaktionszeit | 1 Stunde | 1 Stunde | 1 Stunde | 1 Stunde |
| Besonderheit | - | - | - | - |
| Oberphase | 57.5 g<br>8.0 % 1-Propanol<br>0.9 % Wasser<br>91.1 % Trihexylamin | 62.3 g<br>15.4 % 2-Propanol<br>1.7 % Wasser<br>82.9 % Trihexylamin | 75.6 g<br>10.9 % 1-Propanol<br>0.9 % Wasser<br>88.2 % Tripentylamin | 63.8 g<br>5.7 % 1-Propanol<br>0.5 % Wasser<br>93.8 % Tripentylamin |
| Unterphase | 43.6 g<br>5.9 % Ameisensäure<br>30.3 % 1-Propanol<br>15.6 % Wasser<br>48.3 % Trihexylamin | 37.3 g<br>4.7 % Ameisensäure<br>32.4 % 2-Propanol<br>5.9 % Wasser<br>56.8 % Trihexylamin | 22.9 g<br>3.4 % Ameisensäure<br>41.7 % 1-Propanol<br>28.9 % Wasser<br>26 % Tripentylamin | 38.4 g<br>6.8 % Ameisensäure<br>36.7 % 1-Propanol<br>18.2 % Wasser<br>38,3 % Tripentylamin |
| $k_{Ru}$ ($c_{Ru}$ Oberphase / $c_{Ru}$ Unterphase) | 1.60 | 1.02 | 85 | 2.7 |
| TOF | 252 $h^{-1}$ | 175 $h^{-1}$ | 81 $h^{-1}$ | 250 $h^{-1}$ |
| Reaktionsrate | 0.54 mol $kg^{-1}$ $h^{-1}$ | 0.38 mol $kg^{-1}$ $h^{-1}$ | 0.17 mol $kg^{-1}$ $h^{-1}$ | 0.56 mol $kg^{-1}$ $h^{-1}$ |

**Tabelle 1.2**

| | Beispiel A-5 | Beispiel A-6 | Beispiel A-7 | Beispiel A-8 |
|---|---|---|---|---|
| Tertiäres Amin | 75 g Tripentylamin | 75 g Tripentylamin | 75 g Tripentylamin | 75 g Tripentylamin |
| Polares Lösungsmittel (eingesetzt) | 21.8 g 2-Propanol<br>3.3 g Wasser | 17.8 g 1-Propanol<br>7.3 g Wasser | 17.8 g 1-Propanol<br>7.3 g Wasser | 18.8 g Methanol<br>6.3 q Wasser |
| Katalysator | 0.2 g $[Ru(P^nBu_3)_4(H)_2]$ | 0.2 g $[Ru(P^nBu_3)_4H)_2]$, 0.2 g 1,2-Bis-dicylcohexylphosphinoethan | 0.2 g $[Ru(P^nBu_3)_4(H)_2]$, 0.2 g 1,2-Bis-dicylcohexylphosphinoethan | 0.2 g $[Ru(P^nBu_3)_4(H)_2]$ |
| Aufpressen von $CO_2$ | Mit 20.1 g auf 2.4 MPa abs | Mit 20.2 g auf 2.8 MPa abs | Mit 20.0 g auf 2.5 MPa abs | Mit 20.0 g auf 2.3 MPa abs |
| Aufpressen von $H_2$ | Auf 10.4 MPa abs | Auf 8.1 MPa abs | Auf 10.5 MPa abs | Auf 10.3 MPa |
| Erwärmen | Auf 50°C | Auf 50°C | Auf 50°C | Auf 50°C |
| Druckänderung | Auf 11.0 MPa abs | Auf 8.5 MPa abs | Auf 10.9 MPa abs. | Auf 10.5 MPa |
| Reaktionszeit | 1 Stunde | 1 Stunde | 1 Stunde | 1 Stunde |
| Besonderheit | - | - | - | - |
| Oberphase | 65.7 g<br>11.5 % 2-Propanol<br>1.0 % Wasser<br>87.5 Tripentylamin | 60.6 g<br>5.1 % 1-Propanol<br>94.9 % Tripentylamin | 51.4 g<br>3.9 % 1-Propanol<br>96.1 % Tripentylamin | 60.5 g<br>3.1 % Methanol<br>96.9 % Tripentylamin |
| Unterphase | 35.0 g<br>5.6 % Ameisensäure<br>40.6 % 2-Propanol<br>7.5 % Wasser<br>46.3 % Tripentylamin | 40.8 g<br>7.0 % Ameisensäure<br>36.0 % 1-Propanol<br>17.9 % Wasser<br>39.1 % Tripentylamin | 50.1 g<br>8.4 % Ameisensäure<br>31.5 % 1-Propanol<br>14.6 % Wasser<br>45.5 % Tripentylamin | 40.8 g<br>7.3 % Ameisensäure<br>41.5 % Methanol<br>15.4 % Wasser<br>35.8 % Tripentylamin |
| $k_{Ru}$ ($c_{Ru}$ Oberphase / $c_{Ru}$ Unterphase) | 1.4 | 2.2 | 2.5 | 4.8 |

(fortgesetzt)

| | Beispiel A-5 | Beispiel A-6 | Beispiel A-7 | Beispiel A-8 |
|---|---|---|---|---|
| TOF | 195 $h^{-1}$ | 282 $h^{-1}$ | 413 $h^{-1}$ | 290 $h^{-1}$ |
| Reaktionsrate | 0.43 mol $kg^{-1}$ $h^{-1}$ | 0.61 mol $kg^{-1}$ $h^{-1}$ | 0.90 mol $kg^{-1}$ $h^{-1}$ | 0.64 mol $kg^{-1}$ $h^{-1}$ |

**Tabelle 1.3**

| | Beispiel A-9 | Beispiel A-10 | Beispiel A-11 | Beispiel A-12 |
|---|---|---|---|---|
| Tertiäres Amin | 70 g Trihexylamin | 75 g Tripentylamin | 75 g Tripentylamin | 75 g Trihexylamin |
| Polares Lösungsmittel (eingesetzt) | 15.0 g Ethanol<br>5.0 g Wasser | 21.5 g Methanol<br>3.6 g Wasser | 24.0 g Methanol<br>1.0 g Wasser | 22.0 g Methanol<br>3.0 g Wasser |
| Katalysator | 0.2 g $[Ru(P^nBu_3)_4(H)_2]$ | 0.2 g $[Ru(P^nBu_3)_4(H)_2]$ | 0.16 g $[Ru(P^nOct_3)_4(H)_2]$ | 0.16 g $[Ru(P^nOct_3)_4(H)_2]$, 0.08 g 1,2-Bis(dicyclohexylphosphino)eth an |
| Aufpressen von $CO_2$ | Mit 20.2 g auf 2.5 MPa abs | Mit 20.0 g auf 2.2 MPa abs | Mit 19.9 g auf 2.3 MPa abs | Mit 20.0 g auf 2.5 MPa abs |
| Aufpressen von $H_2$ | Auf 10.9MPa abs | Auf 10.5 MPa abs | Auf 10.3 MPa abs | Auf 10.5 MPa abs |
| Erwärmen | Auf 50°C | Auf 50°C | Auf 50°C | Auf 50°C |
| Druckänderung | Auf 11.8 MPa abs | Auf 10.3 MPa abs | Auf 10.2 MPa abs | Auf 10.6 MPa abs |
| Reaktionszeit | 1 Stunde | 1 Stunde | 1 Stunde | 1 Stunde |
| Besonderheit | - | - | - | - |
| Oberphase | 52.0 g<br>4.6 % Ethanol<br>95.4 % Triehxylamin | 49.5 g<br>2.5 % Methanol<br>77.5 g Tripentylamin | 63.3 g<br>8.4 % Methanol<br>91.6 % Tripentylamin | 46.7 g<br>4.1 % Methanol<br>95.9 % Trihexylamin2 |
| Unterphase | 38.4 g<br>7.3 % Ameisensäure<br>32.8 % Ethanol<br>13.0 % Wasser<br>46.9 % Tripentylamin | 52.8 g<br>8.7 % Ameisensäure<br>38.5 % Methanol<br>6.8 % Wasser<br>46.0 % Tripentylamin | 35.9 g<br>4.5 % Ameisensäure<br>52.1 % Methanol<br>2.8 % Wasser<br>40.7 % Tripentylamin | 54.2 g<br>7.2 % Ameisensäure<br>37.1 % Methanol<br>5.5 % Wasser<br>50.2 % Trihexylamin |
| $k_{Ru}$ ($c_{Ru}$ Oberphase / $c_{Ru}$ Unterphase) | 1.9 | 2.5 | 14.0 | 1.7 |
| TOF | 271 $h^{-1}$ | 446 $h^{-1}$ | 343 $h^{-1}$ | 806 h-$^{1}$ |
| Reaktionsrate | 0.67 mol $kg^{-1}$ $h^{-1}$ | 0.98 mol $kg^{-1}$ $h^{-1}$ | 0.35 mol $kg^{-1}$ $h^{-1}$ | 0.84 mol $kg^{-1}$ $h^{-1}$ |

**Tabelle 1.4**

| | Beispiel A-13 | Beispiel A-14 | Beispiel A-15 | Beispiel A-16 |
|---|---|---|---|---|
| Tertiäres Amin | 75 g Trihexylamin | 75 g Trihexylamin | 75 g Trihexylamin | 75 g Trihexylamin |
| Polares Lösungsmittel (eingesetzt) | 24.0 g Methanol<br>6.7 g Wasser | 25.0 g Ethanol<br>6.0 g Wasser | 25.0 g 1-Propanol<br>6.0 g Wasser | 25.0 g Ethanol<br>8.0 g Wasser |
| Katalysator | 0.18 g [Ru(PnBu3)4(H)2] | 0.16 g [Ru(PnOct3)4(H)2], 0.08 g 1,2-Bis(dicyclohexylphosphino)eth an | 0.16 g [Ru(PnOct3)4(H)2], 0.08 g 1,2-Bis(dicyclohexylphosphino)eth an | 0.16 g [Ru(PnOct3)4(H)2], 0.08 g 1,2-Bis(dicyclohexylphosphino)eth an |
| Aufpressen von CO2 | Mit 20.2 g auf 3.5 MPa abs | Mit 19.9 g auf 2.5 MPa abs | Mit 19.9 g auf 2.5 MPa abs | Mit 19.5 g auf 2.6 MPa abs |
| Aufpressen von H2 | Auf 11.5 MPa abs | Auf 11.5 MPa abs | Auf 10.5 MPa abs | Auf 10.7 MPa abs |
| Erwärmen | Auf 50°C | Auf 50°C | Auf 50°C | Auf 50°C |
| Druckänderung | Auf 11.0 MPa abs | Auf 10.5 MPa abs | Auf 11.3 MPa abs | Auf 11.6 MPa abs |
| Reaktionszeit | 1 Stunde | 1 Stunde | 1 Stunde | 2 Stunden |
| Besonderheit | - | - | | |
| Oberphase | 44.1 g<br>2.7 % Methanol<br>97.3 % Trihexylamin | 60.2 g<br>0.7 % Wasser<br>6.6 % Ethanol<br>92.7 % Trihexylamin | 46.4 g<br>2.0 % Wasser<br>6.2 % 1-Propanol<br>91.8 % Trihexylamin | 48.9 g<br>0.6 % Wasser<br>4.5 % Ethanol<br>94.9 % Trihexylamin |
| Unterphase | 65.9 g<br>7.5 % Ameisensäure<br>10.2 % Wasser<br>34.6 % Methanol<br>47.7 % Trihexylamin | 51.3 g<br>5.3 % Ameisensäure<br>9.3 % Wasser<br>41.0 % Ethanol<br>44.4 % Trihexylamin | 60.6 g<br>5.6 % Ameisensäure<br>8.4 % Wasser<br>36.5 % 1-Propanol<br>49.5 % Trihexylamin | 61.9 g<br>6.0 % Ameisensäure<br>12.4 % Wasser<br>36.8 % Ethanol<br>44.8 % Trihexylamin |
| KRu (cRu Oberphase / cRu Unterphase) | 1.9 | 1.6 | 1.5 | 3.2 |
| TOF | 551 h-1 | 569 h-1 | 726 h-1 | 351 h-1 |

(fortgesetzt)

| | Beispiel A-13 | Beispiel A-14 | Beispiel A-15 | Beispiel A-16 |
|---|---|---|---|---|
| Reaktionsrate | 0.98 mol kg-1 h-1 | 0.53 mol kg-1 h-1 | 0.68 mol kg-1 h-1 | 0.37 mol kg-1 h-1 |

**Tabelle 1.5**

| | Beispiel A-17 |
|---|---|
| Tertiäres Amin | 75 g Trihexylamin |
| Polares Lösungsmittel (eingesetzt) | 25.0 g 1-Propanol<br>8.0 g Wasser |
| Katalysator | 0.16 g [Ru(P$^n$Oct$_3$)$_4$(H)$_2$], 0.08 g 1,2-Bis(dicyclohexylphosphino)eth an |
| Aufpressen von $CO_2$ | Mit 20.3 g auf 2.5 MPa abs |
| Aufpressen von $H_2$ | Auf 10.5 MPa abs |
| Erwärmen | Auf 50°C |
| Druckänderung | Auf 11.4 MPa abs |
| Reaktionszeit | 2 Stunden |
| Besonderheit | - |
| Oberphase | 37.1 g<br>2.6 % Wasser<br>3.9 % 1-Propanol<br>93.5 % Trihexylamin |
| Unterphase | 74.5 g<br>6.2 % Ameisensäure<br>9.4 % Wasser<br>31.5 % 1-Propanol<br>52.9 % Trihexylamin |
| $K_{Ru}$ ($c_{Ru}$ Oberphase / $c_{Ru}$ Unterphase) | 3.9 |
| TOF | 437 $h^{-1}$ |
| Reaktionsrate | 0.45 mol $kg^{-1}$ $h^{-1}$ |

Beispiel B1-3 (Extraktion des Katalysators);

Ein 100 mL Autoklav aus Hastelloy C, ausgestattet mit einem Blattrührer, wurde unter Inertbedingungen mit dem Trialkylamin, polaren Lösungsmittel und dem Katalysator befüllt. Anschließend wurde der Autoklav verschlossen und bei Raumtemperatur wurden $CO_2$ aufgepresst. Nachfolgend wurde $H_2$ aufgepresst und der Reaktor unter Rühren (1000 U/min) erwärmt. Nach der Reaktionszeit wurde der Autoklav abgekühlt und das Reaktionsgemisch entspannt. Es wurde ein zweiphasiges Produktgemisch erhalten, wobei die obere Phase mit dem noch freien tertiären Amin und dem homogenen Katalysator, und die untere Phase mit dem polaren Lösungsmittel und dem gebildeten Ameisensäure/Amin-Addukt angereichert war. Die Unterphase wurde abgetrennt und unter Inertbedingungen dreimal mit der gleichen Menge (Masse an Amin entspricht der Masse der Unterphase) frischem Trialkylamin (10 Minuten bei Raumtemperatur rühren und anschließend Phasen trennen) zur Katalysatorextraktion versetzt. Der Gesamtgehalt an Ameisensäure im Ameisensäure/Amin-Addukt wurde durch Titration mit 0,1 N KOH in MeOH potentiometrisch mit einem "Mettler Toledo DL50"-Titrator bestimmt. Der Rutheniumgehalt wurde per AAS-bestimmt. Die Parameter und Ergebnisse der einzelnen Versuche sind in Tabelle 1.6 wiedergegeben.

Die Beispiele B-1 bis B-3 zeigen, dass beim erfindungsgemäßen Verfahren sich mit tertiärem Amin, welches im Verfahrensschritt 5) erhalten wird, die Menge an Rutheniumkatalysator in der Produktphase extraktiv verringern lässt. Durch weitere Extraktionsschritte bzw. eine kontinuierliche Gegenstromextraktion ließe sich dieser Wert weiter absenken.

**Tabelle 1.6**

| | Beispiel B-1 | Beispiel B-2 | Beispiel B-3 |
|---|---|---|---|
| Tertiäres Amin | 37.5 g Trihexylamin | 37.5 g Tripentylamin | 37.5 g Trihexylamin |

(fortgesetzt)

| | Beispiel B-1 | Beispiel B-2 | Beispiel B-3 |
|---|---|---|---|
| Polares Lösungsmittel (eingesetzt) | 12.0 g Methanol 0.5 g Wasser | 10.0 g Methanol 2.5 g Wasser | 10.0 g Methanol 2.5 g Wasser |
| Katalysator | 0.16 g $[Ru(PnOctyl_3)_4(H)_2]$ | 0.1 g $[Ru(PnButyl_3)_4(H)_2]$ | 0.1 g $[Ru(PnButyl_3)_4(H)_2]$ |
| Aufpressen von $CO_2$ | Auf 1.7 MPa abs | Auf 2.3 MPa abs | Auf 2.5 MPa abs |
| Aufpressen von $H_2$ | Auf 8.0 MPa abs | Auf 8.0 MPa abs | Auf 8.0 MPa abs |
| Erwärmen | 50°C | 50°C | 50°C |
| Reaktionszeit | 1.5 Stunden | 1 Stunde | 1 Stunde |
| Oberphase | 23.3 g | 31.1 g | 22.9 g |
| Unterphase | 26.2 g 6.1 % Ameisensäure | 17.4 g 5.6 % Ameisensäure | 27.5 g 7.2 % Ameisensäure |
| $c_{Ru}$ Oberphase nach Reaktion | 350 ppm | 320 ppm | 250 ppm |
| $c_{Ru}$ Unterphase nach Reaktion | 33 ppm | 80 ppm | 170 ppm |
| $c_{Ru}$ Unterphase nach Extraktion | 21 ppm | 50 ppm | 75 ppm |

Beispiele C1-C4 (Thermische Abtrennung des polaren Lösungsmittels von den Trialkylamin-Solvent-Ameisensäuregemischen, welche nach der Extraktion als Produktphase vorliegen);

Alkohol und Wasser werden von der Produktphase (enthält das Ameisensäure/Amin-Addukt) unter vermindertem Druck mittels eines Rotationsverdampfers abdestilliert. Im Sumpf entsteht ein zweiphasiges Gemisch (Trialkylamin- und Ameisensäure/Amin-Addukt-Phase), die beiden Phasen werden getrennt und der Ameisensäuregehalt der Unterphase wurde durch Titration mit 0,1 N KOH in MeOH potentiometrisch mit einem "Mettler Toledo DL50"-Titrator bestimmt. Der Amin- und Alkoholgehalt wird durch Gaschromatographie bestimmt. Die Parameter und Ergebnisse der einzelnen Versuche sind in Tabelle 1.7 wiedergegeben.

Die Beispiele C-1 bis C-4 zeigen, sich dass beim erfindungsgemäßen Verfahren verschiedene polare Lösungsmittel unter milden Bedingungen von der Produktphase abtrennen lassen, wobei eine ameisensäurereichere Unterphase sowie eine Oberphase, bestehend überwiegend aus tertiärem Amin, anfällt.

**Tabelle 1.7**

| | Beispiel C-1 | Beispiel C-2 | Beispiel C-3 | Beispiel C-4 |
|---|---|---|---|---|
| Einsatzgemisch (Gew.%) | 18.7 g<br>7.2 % Ameisensäure<br>26.4 % 1-Propanol<br>15.5 % Wasser<br>48.3 % Trihexylamin | 19.3 g<br>5.8 % Ameisensäure<br>22.8 % 2-Propanol<br>4.1 % Wasser<br>67.2 % Trihexylamin | 81.8 g<br>7.3 % Ameisensäure<br>41.3 % Methanol<br>15.4 % Wasser<br>35.9 % Tripentylamin | 88.6 g<br>9.2 % Ameisensäure<br>31.4 % Ethanol<br>11.3 % Wasser<br>48.1 % Tripentylamin |
| Ameisensäure : Amin Einsatzgemisch | 1:1.2 | 1 : 2.0 | 1:1 | 1 : 1.1 |
| Druck | 20 mbar | 20 mbar | 200 mbar | 200 mbar |
| Temperatur | 50°C | 50°C | 100°C | 110°C |
| Ameisensäuregehalt Unterphase nach Destillation (Gew.%) | 16.4 % | 18.0% | 23.7 % | 22.7 % |

(fortgesetzt)

| | Beispiel C-1 | Beispiel C-2 | Beispiel C-3 | Beispiel C-4 |
|---|---|---|---|---|
| Ameisensäure : Amin Unterphase nach Destillation (molares Verhältnis) | 1 : 0.76 | 1 : 0.78 | 1 : 0.6 | 1 : 0.56 |
| Wiederfindungsrate Ameisensäure nach Destillation | 95.3 % | 93.7 % | 90.4 % | 95.2 % |

Beispiele D1 und D2 (Thermische Abtrennung des polaren Lösungsmittels von den Trialkylamin-Solvent-Ameisensäuregemischen und Spaltung des Ameisensäure-Amin-Adduktes)

Alkohol und Wasser werden von der Produktphase (enthält das Ameisensäure/Amin-Addukt) unter vermindertem Druck mittels eines Rotationsverdampfers abdestilliert. Im Sumpf entsteht ein zweiphasiges Gemisch (Trialkylamin- und Ameisensäure/Amin-Addukt-Phase) und die beiden Phasen werden getrennt. Die Zusammensetzung des Destillates (enthaltend den Großteil des Methanols sowie des Wassers), der Oberphase (enthaltend das freie Trialkylamin) und der Unterphase (enthaltend das Ameinsäure-Amin-Addukt) wurde durch Gaschromatographie und durch Titration der Ameisensäure gegen 0,1 N KOH in MeOH potentiometrisch mit einem "Mettler Toledo DL50"-Titrator bestimmt. Die Ameisensäure wird dann aus der Unterphase aus dem ersten Schritt in einer Vakuumdestillationsapparatur über eine 10 cm Vigreuxkolonne thermisch vom Trialkylamin abgespalten. Dabei wird nach vollständiger Abspaltung der Ameisensäure ein einphasiger Sumpf, bestehend aus dem reinen Trialkylamin erhalten, welches zur Extraktion des Katalysators und Rückführung in die Hydrierung eingesetzt werden kann. Im Destillat findet sich die Ameisensäure, sowie Restwasser. Die Zusammensetzung des Sumpfes sowie des Destillates wurde durch Gaschromatographie und durch Titration der Ameisensäure mit gegen 0,1 N KOH in MeOH potentiometrisch mit einem "Mettler Toledo DL50"-Titrator bestimmt. Die Parameter und Ergebnisse der einzelnen Versuche sind in Tabelle 1.8 wiedergegeben.

Die Beispiele D-1 und D2 zeigen, sich dass beim erfindungsgemäßen Verfahren verschiedene polare Lösungsmittel unter milden Bedingungen von der Produktphase abtrennen lassen, wobei eine ameisensäurereichere Unterphase sowie eine Oberphase, bestehend überwiegend aus tertiärem Amin. Von dieser ameisensäurereichen Unterphase kann dann die Ameisensäure bei höheren Temperaturen vom Trialkylamin abgespalten werden, wobei das freie Trialklyamin anfällt. Die so gewonnene Ameisnesäure enthält noch etwas Wasser, welches aber durch eine Kolonne mit größerer Trennleistung von der Ameisensäure abgetrennt werden kann. Das sowohl bei der Abtrennung des Lösungsmittels als auch bei der thermischen Spaltung anfallende Trialkylamin kann dazu verwendet werden, den Katalysator im Verfahrensschritt 2) aus dem Produktstrom abzureichern.

**Tabelle 1.8**

| | Beispiel D-1a (Abtrennung des polaren Solvents) | Beispiel D-1b (Spaltung des Ameisensäure-Amin-Adduktes) | Beispiel D-2a (Abtrennung des polaren Solvents | Beispiel D-2b (Spaltung des Ameisensäure-Amin-Adduktes) |
|---|---|---|---|---|
| Einsatzgemisch (Gew.%) | 199.8 g<br>8.9 % Ameisensäure<br>28.4 % Methanol<br>5.6 % Wasser<br>57.1 % Trihexylamin | Unterphase aus D1-a | 199.8 g<br>7.8 % Ameisensäure<br>33.0 % Methanol<br>15.1 % Wasser<br>44.0 % Trihexylamin | Unterphase aus D2-a |
| Ameisensäure : Amin Einsatzgemisch | 1 : 1.1 | 1 : 0.64 | 1:1 | 1 : 0.89 |
| Druck | 200 mbar | 90 mbar | 200 mbar | 90 mbar |
| Temperatur | 120°C | 153°C | 120°C | 153°C |

(fortgesetzt)

| | Beispiel D-1a (Abtrennung des polaren Solvents) | Beispiel D-1b (Spaltung des Ameisensäure-Amin-Adduktes) | Beispiel D-2a (Abtrennung des polaren Solvents | Beispiel D-2b (Spaltung des Ameisensäure-Amin-Adduktes) |
|---|---|---|---|---|
| Unterphase im Sumpf nach Destillation (Gew.%) | 79.8 g<br>22.1 % Ameisensäure<br>1.5 % Wasser<br>76.4 % Trihexylamin | 63.6 g<br>100 % Trihexylamin | 69.4 g<br>14.9 % Ameisensäure<br>6.9 % Wasser<br>78.2 % Trihexylamin | 55.5 g<br>99.7 % Trihexylamin<br>0.3 % Wasser |
| Oberphase im Sumpf nach Destillation | 50.5 g<br>100 % Trihexylamin | einphasig | 32.7 g<br>99.7 % Trihexylamin<br>0.3 % Wasser | einphasig |
| Destillat | 66.6 g<br>0.3 % Ameisensäure<br>81.2 % Methanol<br>18.5 % Wasser | 14.9 g<br>92.1 % Ameisensäure<br>7.9 % Wasser | 93.1 g<br>70.1 % Methanol<br>29.9 % Wasser | 12.9 g<br>85.0 % Ameisensäure<br>15 % Wasser |

Die Zeichnungen zeigen im Einzelnen:

Figur 1 ein Blockdiagramm einer bevorzugten Ausführungsform des erfindungsgemäßen Verfahrens,

Figur 2 ein Blockdiagramm einer weiteren bevorzugten Ausführungsform des erfindungsgemäßen Verfahrens und

Figur 3 ein Blockdiagramm einer zusätzlichen bevorzugten Ausführungsform des erfindungsgemäßen Verfahrens.

Bei der Ausführungsform gemäß Figur 1 werden Kohlendioxid, Strom 1, und Wasserstoff, Strom 2, in den Hydrierreaktor I geführt. Darin werden diese in Gegenwart eines Katalysators enthaltend ein Element aus der 8., 9. oder 10. Gruppe des Periodensystems, eines tertiären Amins und eines polaren Lösungsmittels zu Ameisensäure/Amin-Addukt umgesetzt. Dabei bilden sich zwei Flüssigphasen aus. Die Unterphase ist mit den Ameisensäure/Amin-Addukten sowie dem polaren Lösungsmittel angereichert, die Oberphase mit dem tertiären Amin und im Falle des Einsatzes eines homogenen Katalysators auch mit diesem. Beide Flüssigphasen werden einem Phasenabscheider II zugeführt (Strom 3) und voneinander getrennt. Die Oberphase 4 wird zum Hydrierreaktor I rückgeführt. Die Unterphase 5 wird einer Extraktionsapparatur III zugeführt, in welcher Katalysatorreste mit dem tertiären Amin aus dem Phasentrenngefäß V extrahiert werden. Das tertiäre Amin mit den Katalysatorresten (Strom 6) aus der Extraktionseinheit III wird dann in den Hydrierreaktor I rückgeführt. Die Produktphase 7 aus der Extraktionseinheit III wird der thermischen Abtrenneinheit IV zugeführt, um das polare Lösungsmittel thermisch von den Ameisensäure/Amin-Addukten abzutrennen. Der Strom 7 kann dabei noch zuvor über ein Adsorberbett geführt werden, um letzte Rutheniumspuren aus diesem Strom zu entfernen. Das polare Lösungsmittel, das in der Einheit IV thermisch abgetrennt wird, wird in den Hydrierreaktor I als Strom 8 rückgeführt und das zweiphasige Sumpfgemisch der thermischen Abtrenneinheit IV, bestehend aus den Ameisensäure/Amin-Addukten und dem tertiären Amin (Strom 9) wird dem Phasentrenngefäß V zugeführt. Die Ameisensäure/Amin-Addukte werden im Phasentrenngefäß V abgetrennt und der Destillationseinheit VI als Strom 10 zugeführt, worin dieses in freie Ameisensäure und tertiäres Amin thermisch gespalten wird. Die freie Ameisensäure wird beispielsweise als Kopfprodukt, Strom 12, entfernt. Der zweiphasige Sumpf der Destillationseinheit VI, bestehend aus tertiärem Amin und nicht gespaltenen Ameisensäure/Amin-Addukten, Strom 11, wird wieder dem Phasentrenngefäß V zugeführt. Das tertiäre Amin, welches im Phasentrenngefäß V abgetrennt wird, wird der Extraktionseinheit III zugeführt, um Katalysatorreste zu extrahieren.

Bei der Ausführungsform gemäß Figur 2 werden Kohlendioxid 1 und Wasserstoff 2 in den Hydrierreaktor I geführt. Darin werden diese in Gegenwart eines Katalysators enthaltend ein Element aus der 8., 9. oder 10. Gruppe des Periodensystems, eines tertiären Amins und eines polaren Lösungsmittels zu Ameisensäure/Amin-Addukten umgesetzt. Dabei bilden sich zwei Flüssigphasen aus. Die Unterphase ist mit den Ameisensäure/Amin-Addukten sowie dem polaren Lösungsmittel angereichert, die Oberphase mit dem tertiären Amin und im Falle des Einsatzes eines

homogenen Katalysators auch mit diesem. Beide Flüssigphasen (Strom 3) werden einem Phasenabscheider II zugeführt und voneinander getrennt. Die Oberphase wird zum Hydrierreaktor I rückgeführt (Strom 4). Die Unterphase wird einer Extraktionsapparatur III zugeführt, in welcher Katalysatorreste mit dem tertiären Amin aus dem Phasentrenngefäß V extrahiert werden. Das tertiäre Amin mit den Katalysatorresten aus der Extraktionseinheit III wird dann in den Hydrierreaktor I rückgeführt (Strom 6). Die Produktphase 7 aus der Extraktionseinheit III wird der thermischen Abtrenneinheit IV zugeführt, um das polare Lösungsmittel thermisch von den Ameisensäure/Amin-Addukten abzutrennen. Der Strom 7 kann dabei noch zuvor über ein Adsorberbett geführt werden, um letzte Katalysatorspuren aus diesem Strom zu entfernen. Das polare Lösungsmittel, das in der Einheit IV thermisch abgetrennt wird, wird in den Hydrierreaktor I als Strom 8 rückgeführt und das zweiphasige Sumpfgemisch der thermischen Abtrenneinheit IV, bestehend aus den Ameisensäure/Amin-Addukten und dem tertiären Amin, Strom 9, wird der Destillationseinheit VI als Strom 10 zugeführt. In dieser werden die Ameisensäure/Amin-Addukte in freie Ameisensäure und tertiäres Amin thermisch gespalten. Die freie Ameisensäure wird beispielsweise als Kopfprodukt, Strom 12, entfernt. Der zweiphasige Sumpf der Destillationseinheit VI, Strom 11, bestehend aus tertiärem Amin und nicht gespaltenen Ameisensäure/Amin-Addukten wird dem Phasentrenngefäß V zugeführt. Das tertiäre Amin, welches im Phasentrenngefäß V abgetrennt wird, wird der Extraktionseinheit III als Strom 13 zugeführt, um Katalysatorreste zu extrahieren. Ein Teil von Strom 13 kann auch direkt in den Hydrierreaktor zurückgeführt werden, falls nicht alles Amin für die Extraktion benötigt wird.

Bei der Ausführungsform gemäß Figur 3 werden Kohlendioxid 1 und Wasserstoff 2 in den Hydrierreaktor I geführt. Darin werden diese in Gegenwart eines Katalysators enthaltend ein Element aus der 8., 9. oder 10. Gruppe des Periodensystems, eines tertiären Amins und eines polaren Lösungsmittels zu Ameisensäure/Amin-Addukten umgesetzt. Dabei bilden sich zwei Flüssigphasen aus. Die Unterphase ist mit den Ameisensäure/Amin-Addukten sowie dem polaren Lösungsmittel angereichert, die Oberphase mit dem tertiären Amin und im Falle des Einsatzes eines homogenen Katalysators auch mit diesem. Beide Flüssigphasen werden als Strom 3 einem Phasenscheider II zugeführt und voneinander getrennt. Die Oberphase, Strom 4, wird zum Hydrierreaktor I rückgeführt. Die Unterphase, Strom 5, wird einer Extraktionsapparatur III zugeführt, in welcher Katalysatorreste mit dem tertiären Amin aus den Phasentrenngefäß IV und V extrahiert werden. Das tertiäre Amin mit den Katalysatorresten aus der Extraktionseinheit III wird dann in den Hydrierreaktor I rückgeführt. Die Produktphase 7 aus der Extraktionseinheit III wird der thermischen Abtrenneinheit V zugeführt, um das polare Lösungsmittel thermisch von Ameisensäure/Amin-Addukten abzutrennen. Der Strom 7 kann dabei noch zuvor über ein Adsorberbett geführt werden, um letzte Rutheniumspuren aus diesem Strom zu entfernen. Das polare Lösungsmittel, das in der Einheit IV thermisch abgetrennt wird, wird in den Hydrierreaktor I rückgeführt (Strom 8) und das zweiphasige Sumpfgemisch der Destillationseinheit IV, bestehend aus den Ameisensäure/Amin-Addukten und dem tertiären Amin, Strom 9, wird dem Phasentrenngefäß VII zugeführt. Das tertiäre Amin, welches im Phasentrenngefäß VII abgetrennt wird, wird der Extraktionseinheit III zugeführt. Die Ameisensäure/Amin-Addukte aus dem Phasentrenngefäß VII werden der Destillationseinheit VI zugeführt. In dieser werden die Ameisensäure/Amin-Addukte in freie Ameisensäure und tertiäres Amin thermisch gespalten. Die freie Ameisensäure wird beispielsweise als Kopfprodukt, Strom 12, entfernt. Der zweiphasige Sumpf der Destillationseinheit VI, bestehend aus tertiärem Amin und nicht gespaltenen Ameisensäure/Amin-Addukten, Strom 11, wird dem Phasentrenngefäß V zugeführt. Das tertiäre Amin, welches im Phasentrenngefäß V abgetrennt wird, wird der Extraktionseinheit III als Strom 13 zugeführt, um Katalysatorreste zu extrahieren.

Das erfindungsgemäße Verfahren ermöglicht die Gewinnung von konzentrierter Ameisensäure in hoher Ausbeute und hoher Reinheit durch Hydrierung von Kohlendioxid. Es zeichnet sich insbesondere durch eine besonders einfache Verfahrensführung aus, welche gegenüber dem Stand der Technik ein einfacheres Verfahrenskonzept, einfachere Verfahrensstufen, eine geringere Anzahl an Verfahrensstufen sowie einfachere Apparate aufweist. So wird beispielsweise durch geeignete Wahl des tertiären Amins und des polaren Lösungsmittels im Falle des Einsatzes eines homogenen Katalysators dieser durch Phasentrennung von Ameisensäure/Amin-Addukten getrennt und ohne weitere Aufarbeitungsschritte zum Hydrierreaktor rückgeführt. Die Phasentrennung kann dabei auch unter Druck erfolgen. Aufgrund der zügigen Abtrennung des Katalysators von den gebildeten Ameisensäure/Amin-Addukten wird eine Rückreaktion unter Zersetzung in Kohlendioxid und Wasserstoff unterdrückt. Zudem werden durch die Rückhaltung beziehungsweise Abtrennung des Katalysators aufgrund der Ausbildung zweier Flüssigphasen Verluste an Katalysator und damit Verluste an Edelmetall minimiert. Darüber hinaus kann im Produktstrom verbliebener Katalysator durch die Extraktion mit dem freien Amin aus den thermischen Spalteinheiten nahezu vollständig in den Hydrierreaktor rückgeführt werden, was die Verluste an Edelmetall weiter minimiert und für einen wirtschaftlichen Prozess von sehr großem Vorteil ist und zudem die Zersetzung der Ameisensäure in den thermischen Spalteinheiten weitgehend unterdrückt wird. Ferner ist beim erfindungsgemäßen Verfahren kein aufwändiger, separater Basenaustausch erforderlich, so dass die im Hydrierreaktor gebildeten Ameisensäure/Amin-Addukte direkt zur thermischen Spaltung eingesetzt werden können. Der Einsatz eines niedrigsiedenden polaren Lösungsmittels ermöglicht dessen thermische Abtrennung in einer der thermischen Spaltung der Ameisensäure vorgelagerten Stufe unter milden Bedingungen, wodurch die Veresterung eingesetzter Alkohole und die Zersetzung der Ameisensäure minimiert wird, ein geringerer Energiebedarf nötig ist und eine höhere Reinheit der Ameisensäure erzielt werden kann. Als Folge des einfacheren Verfahrenskonzepts ist die zur Durchführung

des erfindungsgemäßen Verfahrens erforderliche Produktionsanlage gegenüber dem Stand der Technik kompakter im Sinne eines geringeren Platzbedarfs und des Einsatz von wenigeren Apparaten. Sie weist einen geringeren Investitionsaufwand und einen geringeren Energiebedarf auf.

## Patentansprüche

1. Verfahren zur Herstellung von Ameisensäure durch Umsetzung von Kohlendioxid (1) mit Wasserstoff (2) in einem Hydrierreaktor (I) in Gegenwart

   - eines Katalysators, enthaltend ein Element aus der 8., 9 oder 10. Gruppe des Periodensystems,
   - eines tertiären Amins, enthaltend mindestens 12 Kohlenstoffatome pro Molekül, sowie
   - eines polaren Lösungsmittels, enthaltend einen oder mehrere Monoalkohole, ausgewählt aus Methanol, Ethanol, Propanolen und Butanolen sowie Wasser,

   unter Bildung von Ameisensäure/Amin-Addukten als Intermediaten, die anschließend thermisch gespalten werden, wobei ein tertiäres Amin eingesetzt wird, das einen um mindestens 5 °C höheren Siedepunkt als Ameisensäure aufweist und wobei
   sich bei der Umsetzung im Hydrierreaktor (I) zwei Flüssigphasen bilden, und zwar eine Unterphase, enthaltend überwiegend das polare Lösungsmittel, in der die Ameisensäure/Amin-Addukte angereichert vorliegen, sowie eine Oberphase, enthaltend überwiegend das tertiäre Amin, in der der Katalysator angereichert vorliegt,
   **dadurch gekennzeichnet, dass**
   die Aufarbeitung des Austrags (3) aus dem Hydrierreaktor (I) nach den folgenden Verfahrensschritten durchgeführt wird:

   1) Auftrennung der zwei Flüssigphasen aus dem Hydrierreaktor (i) in einem Phasentrenngefäß (II), Rückführung der Oberphase (4) aus dem Phasentrenngefäß (II) in den Hydrierreaktor (I) und Weiterleitung der Unterphase (5) aus dem Phasentrenngefäß (II) in eine Extraktionsapparatur (III), worin
   2) Reste an Katalysator mit demselben tertiären Amin, das in der Hydrierung eingesetzt wurde, extrahiert und das mit Katalysator beladene tertiäre Amin (6) in den Hydrierreaktor (I) recycliert wird, und der katalysatorfreie Strom von mit den Ameisensäure/Amin-Addukten beladenem polaren Lösungsmittel (7) in eine Destillationseinheit (IV) weitergeleitet wird, worin
   3) das polare Lösungsmittel als Kopfstrom (8) abgetrennt und in den Hydrierreaktor (I) recycliert wird, unter Erhalt eines Stromes (9), der
   4) in einem Phasentrenngefäß (V) in eine Oberphase enthaltend überwiegend das tertiäre Amin und eine Unterphase, enthaltend überwiegend die Ameisensäure/Amin-Addukte, aufgetrennt wird, wobei
   5) die Unterphase aus dem Phasentrenngefäß (V) einer thermischen Spalteinheit (VI) zugeführt, und darin in einen das tertiäre Amin enthaltenden Strom (11), der in das Phasentrenngefäß (V) zurückgeführt wird und in reine Ameisensäure (12) gespalten wird, und wobei
   ein das tertiäre Amin enthaltender Strom (13) aus dem Phasentrenngefäß (V) in die Extraktionsapparatur (III) als selektives Lösungsmittel für den Katalysator geleitet wird.

2. Verfahren nach Anspruch 1, **dadurch gekennzeichnet, dass** man als tertiäres Amin ein Amin der allgemeinen Formel (Ia)

   $$NR^1R^2R^3 \qquad (Ia)$$

   einsetzt, in der die Reste $R^1$ bis $R^3$ gleich oder verschieden sind und unabhängig voneinander einen unverzweigten oder verzweigten, acyclischen oder cyclischen, aliphatischen, araliphatischen oder aromatischen Rest mit jeweils 1 bis 6 Kohlenstoffatomen darstellen, wobei einzelne Kohlenstoffatome unabhängig voneinander auch durch eine Heterogruppe ausgewählt aus der Gruppe -O- und >N- substituiert sein können sowie zwei oder alle drei Reste unter Bildung einer mindestens jeweils vier Atome umfassenden Kette auch miteinander verbunden sein können, mit der Bedingung, dass das tertiäre Amin mindestens 12 Kohlenstoffatome pro Molekül enthält.

3. Verfahren nach Anspruch 2, **dadurch gekennzeichnet, dass** man als tertiäres Amin ein Amin der allgemeinen Formel (la) einsetzt, in der die Reste $R^1$ bis $R^3$ unabhängig voneinander ausgewählt sind aus der Gruppe $C_1$- bis $C_{12}$-Alkyl, $C_5$- bis $C_8$-Cycloalkyl, Benzyl und Phenyl.

4. Verfahren nach Anspruch 2, **dadurch gekennzeichnet, dass** man als tertiäres Amin ein gesättigtes Amin der allgemeinen Formel (Ia) einsetzt.

5. Verfahren nach Anspruch 2, **dadurch gekennzeichnet, dass** man als tertiäres Amin ein Amin der allgemeinen Formel (Ia) einsetzt, in der die Reste $R^1$ bis $R^3$ unabhängig voneinander ausgewählt sind aus $C_5$- und $C_6$-Alkyl.

6. Verfahren nach einem der Ansprüche 1 bis 5, **dadurch gekennzeichnet, dass** man als polares Lösungsmittel Methanol und/oder Ethanol einsetzt.

7. Verfahren nach einem der Ansprüche 1 bis 6, **dadurch gekennzeichnet, dass** das polare Lösungsmittel bis zu 50 Gew.-% Wasser enthält.

8. Verfahren nach einem der Ansprüche 1 bis 7, **dadurch gekennzeichnet, dass** der Katalysator ein homogener Katalysator ist.

9. Verfahren nach Anspruch 8, **dadurch gekennzeichnet, dass** der homogene Katalysator eine metallorganische Komplexverbindung ist, enthaltend ein Element aus der 8., 9. oder 10. Gruppe des Periodensystems und mindestens eine Phosphingruppe mit mindestens einem unverzweigten oder verzweigten, acyclischen oder cyclischen aliphatischen Rest mit 1 bis 12 Kohlenstoffatomen, wobei einzelne Kohlenstoffatome auch durch >P- substituiert sein können.

10. Verfahren nach einem der Ansprüche 1 bis 9, **dadurch gekennzeichnet, dass** die Umsetzung im Hydrierreaktor (I) bei einer Temperatur im Bereich zwischen 20 und 200 °C und bei einem Druck im Bereich zwischen 0,2 und 30 MPa abs durchgeführt wird.

11. Verfahren nach Anspruch 10, **dadurch gekennzeichnet, dass** der Druck im Hydrierreaktor (I) und im Phasentrenngefäß (II) gleich oder annähernd gleich ist.

12. Verfahren nach Anspruch 10, **dadurch gekennzeichnet, dass** die Temperatur im Hydrierreaktor (I) und im Phasentrenngefäß (II) gleich oder annähernd gleich ist.

## Claims

1. A process for preparing formic acid by reaction of carbon dioxide (1) with hydrogen (2) in a hydrogenation reactor (I) in the presence of

   - a catalyst comprising an element of group 8, 9 or 10 of the Periodic Table,
   - a tertiary amine comprising at least 12 carbon atoms per molecule and
   - a polar solvent comprising one or more monoalcohols selected from among methanol, ethanol, propanols and butanols and also water,

   to form formic acid/amine adducts as intermediates which are subsequently thermally dissociated,
   where a tertiary amine which has a boiling point at least 5°C higher than that of formic acid is used and two liquid phases are formed in the reaction in the hydrogenation reactor (I), namely a lower phase which comprises predominantly the polar solvent and in which the formic acid/amine adducts are present in enriched form and an upper phase which comprises predominantly the tertiary amine and in which the catalyst is present in enriched form, wherein
   the work-up of the output (3) from the hydrogenation reactor (I) is carried out by the following process steps:

   1) separation of the two liquid phases from the hydrogenation reactor (i) in a phase separation vessel (II), recirculation of the upper phase (4) from the phase separation vessel (II) to the hydrogenation reactor (I) and passing-on of the lower phase (5) from the phase separation vessel (II) to an extraction apparatus (III) in which
   2) residues of catalyst are extracted with the same tertiary amine which was used in the hydrogenation and the catalyst-laden tertiary amine (6) is recycled to the hydrogenation reactor (I) and the catalyst-free stream of polar solvent (7) loaded with the formic acid/amine adducts is passed on to a distillation unit (IV) in which
   3) the polar solvent is separated off as overhead stream (8) and recycled to the hydrogenation reactor (I) to give a stream (9) which

4) is separated in a phase separation vessel (V) into an upper phase comprising predominantly the tertiary amine and a lower phase comprising predominantly the formic acid/amine adducts, where
5) the lower phase from the phase separation vessel (V) is fed to a thermal dissociation unit (VI) and dissociated therein into a stream (11) which comprises the tertiary amine and is recirculated to the phase separation vessel (V) and pure formic acid (12) and
a stream (13) comprising the tertiary amine is conveyed from the phase separation vessel (V) into the extraction apparatus (III) as selective solvent for the catalyst.

**2.** The process according to claim 1, wherein an amine of the general formula (Ia)

$$NR^1R^2R^3 \qquad (a)$$

where the radicals $R^1$ to $R^3$ are identical or different and are each, independently of one another, an unbranched or branched, acyclic or cyclic, aliphatic, araliphatic or aromatic radical having in each case from 1 to 6 carbon atoms, where individual carbon atoms can also be substituted, independently of one another, by a hetero group selected from the group consisting of -O- and >N- or two or all three radicals can also be joined to one another to form a chain comprising at least four atoms in each case, is used as tertiary amine, with the proviso that the tertiary amine comprises at least 12 carbon atoms per molecule.

**3.** The process according to claim 2, wherein an amine of the general formula (Ia) in which the radicals $R^1$ to $R^3$ are selected independently from the group consisting of $C_1$-$C_{12}$-alkyl, $C_5$-$C_8$-cycloalkyl, benzyl and phenyl is used as tertiary amine.

**4.** The process according to claim 2, wherein a saturated amine of the general formula (Ia) is used as tertiary amine.

**5.** The process according to claim 2, wherein an amine of the general formula (Ia) in which the radicals $R^1$ to $R^3$ are selected independently from $C_5$ and $C_6$-alkyl is used as tertiary amine.

**6.** The process according to any of claims 1 to 5, wherein methanol and/or ethanol are used as polar solvent.

**7.** The process according to any of claims 1 to 6, wherein the polar solvent comprises up to 50% by weight of water.

**8.** The process according to any of claims 1 to 7, wherein the catalyst is a homogeneous catalyst.

**9.** The process according to claim 8, wherein the homogeneous catalyst is a metal-organic complex comprising an element of group 8, 9 or 10 of the Periodic Table and at least one phosphine group having at least one unbranched or branched, acyclic or cyclic aliphatic radical having from 1 to 12 carbon atoms, with individual carbon atoms also being able to be substituted by > P-.

**10.** The process according to any of claims 1 to 9, wherein the reaction in the hydrogenation reactor (I) is carried out at a temperature in the range from 20 to 200°C and a pressure in the range from 0.2 to 30 MPa abs.

**11.** The process according to claim 10, wherein the pressure in the hydrogenation reactor (I) and in the phase separation vessel (II) is the same or approximately the same.

**12.** The process according to claim 10, wherein the temperature in the hydrogenation reactor (I) and in the phase separation vessel (II) is the same or approximately the same.

## Revendications

**1.** Procédé pour la production d'acide formique par mise en réaction de dioxyde de carbone (1) avec de l'hydrogène (2) dans un réacteur d'hydrogénation (I) en présence

- d'un catalyseur, contenant un élément du 8$^e$, 9$^e$ ou 10$^e$ groupe du système périodique,
- d'une amine tertiaire, comportant au moins 12 atomes de carbone par molécule, ainsi que
- d'un solvant polaire, contenant un ou plusieurs monoalcools, choisis parmi le méthanol, l'éthanol, les propanols et les butanols, ainsi que d'eau,

avec formation d'adduits acide formique/amine en tant que composés intermédiaires, qui sont ensuite scindés par voie thermique,
dans lequel on utilise une amine tertiaire qui présente un point d'ébullition supérieur d'au moins 5 °C à celui de l'acide formique, et dans lequel
lors de la réaction dans le réacteur d'hydrogénation (I) il se forme deux phases liquides, à savoir une phase inférieure, contenant essentiellement le solvant polaire, dans lequel les adduits acide formique/amine sont présents en étant concentrés, ainsi qu'une phase supérieure, contenant essentiellement l'amine tertiaire, dans laquelle le catalyseur se trouve en étant concentré,
**caractérisé en ce que**
le traitement final du courant de décharge (3) provenant du réacteur d'hydrogénation (I) est effectué selon les étapes de processus suivantes :

1) séparation des deux phases liquides provenant du réacteur d'hydrogénation (I) dans un récipient de séparation de phases (II), recyclage de la phase supérieure (4) provenant du récipient de séparation de phases (II) dans le réacteur d'hydrogénation (I) et transfert de la phase inférieure (5) provenant du récipient de séparation de phases (II) dans un appareil d'extraction (III), dans lequel
2) les résidus de catalyseur sont extraits avec la même amine tertiaire que celle ayant été utilisée dans l'hydrogénation, et l'amine tertiaire (6) chargée de catalyseur est recyclée dans le réacteur d'hydrogénation (I), et le courant, privé de catalyseur, de solvant polaire (7) chargé des adduits acide formique/amine, est transféré dans une unité de distillation (IV), dans laquelle
3) le solvant polaire est séparé en tant que courant de tête (8) et est recyclé dans le réacteur d'hydrogénation (I), avec obtention d'un courant (9), qui
4) dans un récipient de séparation de phases (V) est fractionné en une phase supérieure contenant essentiellement l'amine tertiaire et une phase inférieure, contenant essentiellement les adduits acide formique/amine,
5) la phase inférieure provenant du récipient de séparation de phases (V) étant envoyée à une unité de coupure thermique (VI) et dans celle-ci scindée en un courant (11) contenant l'amine tertiaire, qui est renvoyé dans le récipient de séparation de phases (V) et est scindé en acide formique pur (12), et
un courant (13) contenant l'amine tertiaire, provenant du récipient de séparation de phases (V) étant envoyé dans l'appareil d'extraction (III) en tant que solvant sélectif pour le catalyseur.

2. Procédé selon la revendication 1, **caractérisé en ce qu'**on utilise comme amine tertiaire une amine de formule générale (Ia)

$$NR^1R^2R^3 \qquad (Ia)$$

dans laquelle les radicaux $R^1$ à $R^3$ sont identiques ou différents et représentent chacun indépendamment un radical aliphatique acyclique ou cyclique, ramifié ou non ramifié, araliphatique ou aromatique, ayant chaque fois de 1 à 6 atomes de carbone, des atomes de carbone individuels pouvant, indépendamment les uns des autres, être également remplacés par un hétéroatome choisi dans le groupe constitué par -O- et >N- et deux ou tous les trois radicaux pouvant également être liés entre eux avec formation d'une chaîne comprenant au moins chaque fois quatre atomes, étant entendu que l'amine tertiaire contient au moins 12 atomes de carbone par molécule.

3. Procédé selon la revendication 2, **caractérisé en ce qu'**on utilise comme amine tertiaire une amine de formule générale (Ia), dans laquelle les radicaux $R^1$ à $R^3$ sont choisis chacun indépendamment dans le groupe constitué par des groupes alkyle en $C_1$-$C_{12}$, cycloalkyle en $C_5$-$C_8$, benzyle et phényle.

4. Procédé selon la revendication 2, **caractérisé en ce qu'**on utilise comme amine tertiaire une amine saturée de formule générale (Ia).

5. Procédé selon la revendication 2, **caractérisé en ce qu'**on utilise comme amine tertiaire une amine de formule générale (Ia), dans laquelle les radicaux $R^1$ à $R^3$ sont choisis chacun indépendamment parmi des groupes alkyle en $C_5$ et $C_6$.

6. Procédé selon l'une quelconque des revendications 1 à 5, **caractérisé en ce qu'**on utilise comme solvant polaire le méthanol et/ou l'éthanol.

7. Procédé selon l'une quelconque des revendications 1 à 6, **caractérisé en ce que** le solvant polaire contient jusqu'à 50 % en poids d'eau.

**8.** Procédé selon l'une quelconque des revendications 1 à 7, **caractérisé en ce que** le catalyseur est un catalyseur homogène.

**9.** Procédé selon la revendication 8, **caractérisé en ce que** le catalyseur homogène est un composé complexe organométallique, contenant un élément du 8$^e$, 9$^e$ ou 10$^e$ groupe du système périodique et au moins un groupe phosphino comportant au moins un radical aliphatique, acyclique ou cyclique, ramifié ou non ramifié, ayant de 1 à 12 atomes de carbone, dans lequel des atomes de carbone individuels peuvent également être remplacés par >P-.

**10.** Procédé selon l'une quelconque des revendications 1 à 9, **caractérisé en ce que** la réaction dans le réacteur d'hydrogénation (I) est effectuée à une température dans la plage comprise entre 20 et 200 °C et sous une pression dans la plage comprise entre 0,2 et 30 MPa (absolue).

**11.** Procédé selon la revendication 10, **caractérisé en ce que** la pression dans le réacteur d'hydrogénation (I) et dans le récipient de séparation de phases (II) est la même ou approximativement la même.

**12.** Procédé selon la revendication 10, **caractérisé en ce que** la température dans le réacteur d'hydrogénation (I) et dans le récipient de séparation de phases (II) est la même ou approximativement la même.

Figur 1

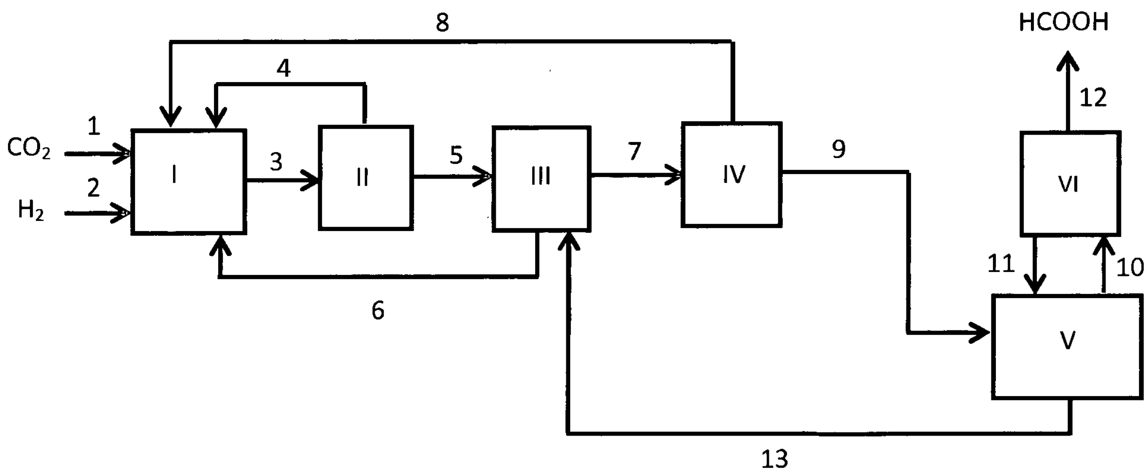

Figur 2

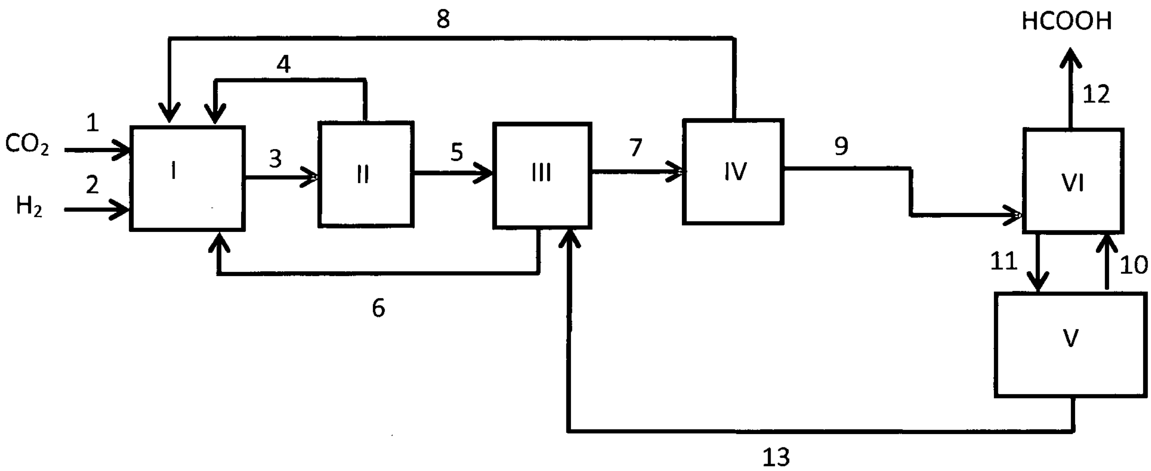

Figur 3

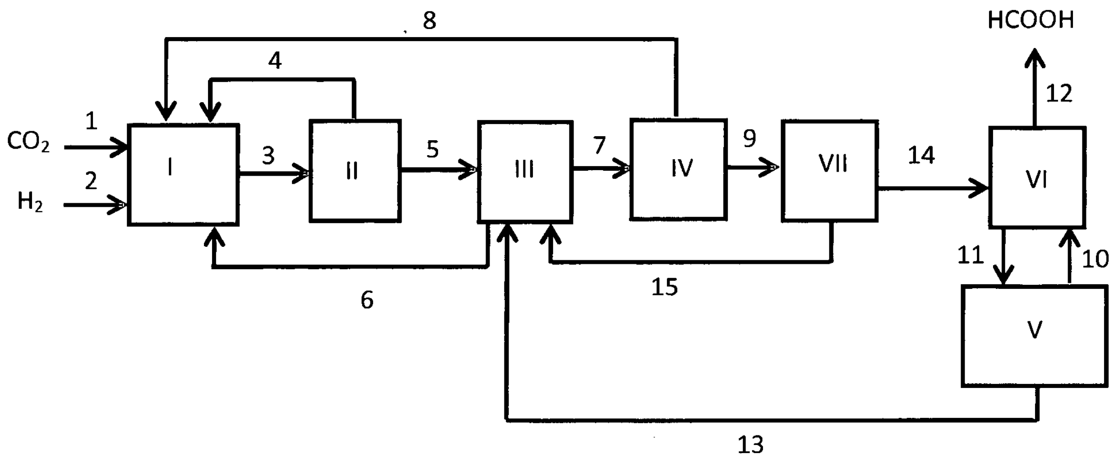

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 68920933 T2
- WO 2008116799 A
- EP 0095321 A
- EP 0151510 A
- EP 0181078 A
- EP 0126524 A
- EP 0329337 A
- EP 0357243 A
- DE 4431233 A
- WO 2006021411 A
- EP 181078 B1
- EP 357243 B1


### In der Beschreibung aufgeführte Nicht-Patentliteratur


- **W. LEITNER.** *Angewandte Chemie,* 1995, vol. 107, 2391-2405
- **P. G. JESSOP ; T. IKARIYA ; R. NOYORI.** *Chemical Reviews,* 1995, vol. 95, 259-272
- **C. FELLAY ; N. YAN ; P.J. DYSON ; G. LAURENCZY.** *Chem. Eur. J.,* 2009, vol. 15, 3752-3760
- **C. FELLAY ; P.J. DYSON ; G. LAURENCZY.** *Angew. Chem.,* 2008, vol. 120, 4030-4032
- **B. LOGES ; A. BODDIEN ; H. JUNGE ; M. BELLER.** *Angew. Chem.,* 2008, vol. 120, 4026-4029
- **F. JOÓ.** *ChemSusChem,* 2008, vol. 1, 805-808
- **S. ENTHALER.** *ChemSusChem,* 2008, vol. 1, 801-804
- **S. FUKUZUMI ; T. KOBAYASHI ; T. SUENOBU.** *ChemSusChem,* 2008, vol. 1, 827-834
- **A. BODDIEN ; B. LOGES ; H. JUNGE ; M. BELLER.** *ChemSusChem,* 2008, vol. 1, 751-758
- **P.G. JESSOP ; T. IKARIYA ; R. NOYORI.** *Chem. Rev.,* 1995, vol. 95, 259-272
- **P.G. JESSOP ; F. JOÓ ; C.C. TAI.** *Coord. Chem. Rev.,* 2004, vol. 248, 2425-2442
- Homogeneous Hydrogenation of Carbon Dioxide. **P.G. JESSOP.** The Handbook of Homogeneous Hydrogenation. Wiley-VCH, 2007, vol. 1, 489-511
- Liquid-Liquid Extraction. **E. MÜLLER et al.** Ullmann's Encylcopedia of Industrial Chemistry. Wiley-VCH Verlag GmbH & Co. KGaA, 2005
- **J.F. HARTWIG.** Organotransition Metal Chemistry. Universtiy Science Books, 2010, 545